# EUROPEAN PATENT APPLICATION

(11) **EP 4 640 708 A1**
(43) Date of publication of application: **29.10.2025**
(21) Application number: 23906117.9
(22) Date of filing: 22.12.2023
(51) Int. Cl.: C07K 16/28, C12N 15/12, C12N 15/13, A61K 39/395, A61P 35/00, A61P 35/04

(54) **NOVEL ANTI-GPRC5D ANTIBODY**

(30) Priority: 23.12.2022 CN 202211667528
(71) Applicant: Chimagen Biosciences, Ltd, Shanghai 201203 (CN)
(72) Inventor: ZHOU, Zhenhao, Shanghai 201203 (CN); ZHANG, Jie, Shanghai 201203 (CN); WANG, Gong, Shanghai 201203 (CN); WANG, Xiaoqing, Shanghai 201203 (CN); ZHANG, Xiaoqin, Shanghai 201203 (CN); WANG, Qi, Shanghai 201203 (CN)
(74) Representative: Simmons & Simmons LLP (Munich)
(86) International application number: PCT/CN2023/141146
(87) International publication number: WO 2024/131962

(57) **Abstract**

The present invention provides an anti-GPRC5D antibody or an antigen-binding fragment thereof, an isolated polynucleotide encoding same, a pharmaceutical composition comprising same, and a use thereof.

## Description

### FIELD OF THE INVENTION

The present application generally relates to a novel anti-GPRC5D antibody and use thereof.

### BACKGROUND

G-protein-coupled receptor family C group 5 member D (GPRC5D) is a 7-transmembrane protein and an orphan receptor. It is reported that the overexpression of GPRC5D is observed in patients with multiple myeloma. In particular, its high expression has a significant correlation with the poor results of disease and treatment. In view of the high specific expression of GPRC5D protein on tumor cells, GPRC5D has a high potential to become a next hot candidate target for the treatment of multiple myeloma.

Therefore, there is an urgent need in this field to develop a novel anti-GPRC5D antibody.

### SUMMARY OF THE INVENTION

Throughout the present application, the articles "a/an" and "the" are used herein to refer to one or more (i.e., at least one) grammatical object following the article. For example, "an antibody" means one antibody or more than one antibody.

The present application provides an anti-GPRC5D antibody (for example, anti-human GPRC5D antibody) or an antigen binding fragment thereof, an isolated polynucleotide encoding the same, a pharmaceutical composition comprising the same, and use thereof.

In an aspect, the present application provides an anti-GPRC5D antibody or an antigen binding fragment thereof, which comprises three heavy chain complementarity determining regions HCDR1, HCDR2 and HCDR3, and three light chain complementarity determining regions LCDR1, LCDR2 and LCDR3. The heavy chain complementarity determining regions are identical to three heavy chain complementarity determining regions included in a heavy chain variable region (V_{H}) as shown in SEQ ID NO: 13, and the light chain complementarity determining regions are identical to three light chain complementarity determining regions included in a light chain variable region (V_{L}) as shown in SEQ ID NO: 12.

In some embodiments, the anti-GPRC5D antibody or antigen binding fragment thereof includes three heavy chain complementarity determining regions HCDR1, HCDR2 and HCDR3, and three light chain complementarity determining regions LCDR1, LCDR2 and LCDR3. a) The HCDR1 includes an amino acid sequence selected from the group consisting of SEQ ID NO: 4 or a variant thereof having no more than 3, 2 or 1 amino acid substitution, or SEQ ID NO: 9 or a variant thereof having no more than 3, 2 or 1 amino acid substitution. b) The HCDR2 includes an amino acid sequence selected from the group consisting of SEQ ID NO: 5 or a variant thereof having no more than 3, 2 or 1 amino acid substitution, or SEQ ID NO: 10 or a variant thereof having no more than 3, 2 or 1 amino acid substitution. c) The HCDR3 includes an amino acid sequence selected from the group consisting of SEQ ID NO: 6 or a variant thereof having no more than 3, 2 or 1 amino acid substitution, or SEQ ID NO: 11 or a variant thereof having no more than 3, 2 or 1 amino acid substitution. d) The LCDR1 includes an amino acid sequence selected from the group consisting of SEQ ID NO: 1 or a variant thereof having no more than 3, 2 or 1 amino acid substitution, or SEQ ID NO: 7 or a variant thereof having no more than 3, 2 or 1 amino acid substitution. e) The LCDR2 includes an amino acid sequence selected from the group consisting of SEQ ID NO: 2 or a variant thereof having no more than 3, 2 or 1 amino acid substitution, or SEQ ID NO: 8 or a variant thereof having no more than 3, 2 or 1 amino acid substitution. f) The LCDR3 includes an amino acid sequence selected from the group consisting of SEQ ID NO: 3 or a variant thereof having no more than 3, 2 or 1 amino acid substitution.

In some embodiments, a) the HCDR1 comprises the amino acid sequence as shown in SEQ ID NO: 4 or a variant thereof having no more than 3, 2 or 1 amino acid substitution, the HCDR2 comprises the amino acid sequence as shown in SEQ ID NO: 5 or a variant thereof having no more than 3, 2 or 1 amino acid substitution, the HCDR3 comprises the amino acid sequence as shown in SEQ ID NO: 6 or a variant thereof having no more than 3, 2 or 1 amino acid substitution, the LCDR1 comprises the amino acid sequence as shown in SEQ ID NO: 1 or a variant thereof having no more than 3, 2 or 1 amino acid substitution, the LCDR2 comprises the amino acid sequence as shown in SEQ ID NO: 2 or a variant thereof having no more than 3, 2 or 1 amino acid substitution, and the LCDR3 comprises the amino acid sequence as shown in SEQ ID NO: 3 or a variant thereof having no more than 3, 2 or 1 amino acid substitution; or the HCDR1 includes the amino acid sequence as shown in SEQ ID NO: 9 or a variant thereof having no more than 3, 2 or 1 amino acid substitution, the HCDR2 comprises the amino acid sequence as shown in SEQ ID NO: 10 or a variant thereof having no more than 3, 2 or 1 amino acid substitution, the HCDR3 comprises the amino acid sequence as shown in SEQ ID NO: 11 or a variant thereof having no more than 3, 2 or 1 amino acid substitution, the LCDR1 comprises the amino acid sequence as shown in SEQ ID NO: 7 or a variant thereof having no more than 3, 2 or 1 amino acid substitution, the LCDR2 comprises the amino acid sequence as shown in SEQ ID NO: 8 or a variant thereof having no more than 3, 2 or 1 amino acid substitution, and the LCDR3 comprises the amino acid sequence as shown in SEQ ID NO: 3 or a variant thereof having no more than 3, 2 or 1 amino acid substitution.

In some embodiments, the heavy chain variable region includes the amino acid sequence as shown in SEQ ID NO: 13 or a variant thereof having no more than 3, 2 or 1 amino acid substitution, and the light chain variable region includes the amino acid sequence as shown in SEQ ID NO: 12 or a variant thereof having no more than 3, 2 or 1 amino acid substitution.

In some embodiments, the antibody or antigen binding fragment thereof is humanized.

In some embodiments, the antibody or antigen binding fragment thereof includes a heavy chain variable region (V_{H}) and/or a light chain variable region (V_{L}), where a) the heavy chain variable region includes an amino acid sequence selected from the group consisting of SEQ ID NO: 15, SEQ ID NO: 17, SEQ ID NO: 19, and SEQ ID NO: 21, or a variant thereof having no more than 3, 2 or 1 amino acid substitution; and b) the light chain variable region includes an amino acid sequence selected from the group consisting of SEQ ID NO: 14, SEQ ID NO: 16, SEQ ID NO: 18, and SEQ ID NO: 20, or a variant thereof having no more than 3, 2 or 1 amino acid substitution.

In some embodiments, the antibody or antigen binding fragment thereof includes a heavy chain variable region (V_{H}) and/or a light chain variable region (V_{L}), in which a) the heavy chain variable region includes the amino acid sequence as shown in SEQ ID NO: 15 or a variant thereof having no more than 3, 2 or 1 amino acid substitution, and the light chain variable region includes the amino acid sequence as shown in SEQ ID NO: 14 or a variant thereof having no more than 3, 2 or 1 amino acid substitution; b) the heavy chain variable region includes the amino acid sequence as shown in SEQ ID NO: 17 or a variant thereof having no more than 3, 2 or 1 amino acid substitution, and the light chain variable region includes the amino acid sequence as shown in SEQ ID NO: 16 or a variant thereof having no more than 3, 2 or 1 amino acid substitution; c) the heavy chain variable region includes the amino acid sequence as shown in SEQ ID NO: 19 or a variant thereof having no more than 3, 2 or 1 amino acid substitution, and the light chain variable region includes the amino acid sequence as shown in SEQ ID NO: 18 or a variant thereof having no more than 3, 2 or 1 amino acid substitution; or the heavy chain variable region includes the amino acid sequence as shown in SEQ ID NO: 21 or a variant thereof having no more than 3, 2 or 1 amino acid substitution, and the light chain variable region includes the amino acid sequence as shown in SEQ ID NO: 20 or a variant thereof having no more than 3, 2 or 1 amino acid substitution, where the variant still retains the specific binding affinity to GPRC5D.

In some embodiments, the amino acid substitution does not occur in the CDR region.

In some embodiments, the antibody or antigen binding fragment thereof is a diabody, Fab, Fab', F(ab')2, Fd, a Fv fragment, a disulfide-stabilized Fv fragment (dsFv), (dsFv)2, a bispecific dsFv (dsFv-dsFv'), a disulfide-stabilized diabody (ds diabody), a single-chain antibody molecule (scFv), or a scFv dimer (a divalent diabody).

In some embodiments, the antibody or antigen binding fragment thereof further includes an immunoglobulin constant region, optionally a human immunoglobulin constant region, or optionally a human IgG constant region.

In some embodiments, the antibody or antigen binding fragment thereof is a humanized monoclonal antibody. In some embodiments, the antibody or antigen binding fragment thereof is bispecific or multi-specific.

In some embodiments, the IgG constant region is derived from IgG1, IgG2, IgG3 or IgG4.

In some embodiments, the constant region comprises one or more amino acid residue substitutions or modifications which lead to increased CDC or ADCC relative to wild-type constant region.

In another aspect, the present application provides a nucleic acid including a nucleotide sequence encoding the antibody or antigen binding fragment thereof as described in the present application.

In another aspect, the present application provides a vector including the nucleic acid as described in the present application.

In another aspect, the present application provides a host cell including the nucleic acid as described in the present application or the vector as described in the present application.

In another aspect, the present application provides a chimeric antigen receptor (CAR), comprising an antigen binding domain, a transmembrane domain, and an immune cell signal transduction domain, where the antigen binding domain specifically binds to GPRC5D and comprises the antigen binding fragment of the anti-GPRC5D antibody provided in the present application.

In another aspect, the present application provides a nucleic acid encoding the chimeric antigen receptor (CAR) as described in the present application.

In another aspect, the present application provides a cell comprising the nucleic acid sequence provided in the present application that encodes the chimeric antigen receptor (CAR) as described in the present application.

In another aspect, the present application provides a genetically modified cell expressing the chimeric antigen receptor (CAR) as described in the present application.

In another aspect, the present application provides a conjugate, including the antibody or antigen binding fragment as described in the present application and a payload conjugated to the antibody or antigen binding fragment, where the payload is selected from the group consisting of a radioactive label, a fluorescent label, an enzyme substrate label, an affinity purification tag, a tracer molecule, an anticancer drug and a cytotoxic molecule.

In another aspect, the present application provides a pharmaceutical composition, which comprises the antibody or antigen-binding fragment thereof as described in the present application, the nucleic acid as described in the present application, the vector as described in the present application, the conjugate as described in the present application, the nucleic acid having a nucleic acid sequence encoding the chimeric antigen receptor (CAR) of the present application as described in the present application, the cell expressing the chimeric antigen receptor (CAR) of the present application as described in the present application, and a pharmaceutically acceptable carrier.

In another aspect, the present application provides a method for treating or preventing a disease, a disorder, or a condition, including administering a therapeutically effective amount of the antibody or antigen binding fragment as described in the present application, the pharmaceutical composition as described in the present application, the conjugate as described in the present application or the composition as described in the present application to a subject in need thereof.

The disease, the disorder or the condition is selected from the group consisting of cancers, autoimmune diseases, and inflammations.

In another aspect, the present application provides a method for stimulating an immune cell (such as T cell or NK cell)-mediated immune response to cells or tissues expressing GPRC5D in a mammal, the method comprising administering an effective amount of genetically modified cells to the mammal to express the CAR in the present application.

In another aspect, the present application provides a method for treating a mammal having a GPRC5D-related disease or condition, the method comprising administering an effective amount of the cell provided in the present application to the mammal, to treat the mammal.

In one embodiment, the cell is autologous T cells or autologous NK cells.

### BRIEF DESCRIPTION OF ACCOMPANYING DRAWINGS

Fig. 1 shows the binding of an GPRC5D antibody (ch-72C7 and GC5B596) to HEK293 cells expressing hGPRC5D in an FACS assay.
Fig. 2 shows the binding of an GPRC5D antibody (ch-72C7 and GC5B596) to CHOS cells expressing hGPRC5D in an FACS assay.
Fig. 3 shows the binding of an GPRC5D antibody (ch-72C7 and GC5B596) to natural MM.1R cells expressing GPRC5D in an FACS assay.
Fig. 4 shows the binding of an GPRC5D antibody (ch-72C7 and GC5B596) to natural NCI-H929 cells expressing GPRC5D in an FACS assay.
Fig. 5 shows the binding of an GPRC5D antibody (ch-72C7 and GC5B596) to natural RPMI-8226 cells expressing GPRC5D in an FACS assay.
Fig. 6 shows the cytotoxicity of an GPRC5D antibody (ch-72C7 and GC5B596) on natural NCI-H929 cells expressing GPRC5D in an ADCC activity assay.
Fig. 7 shows the cytotoxicity of an GPRC5D antibody (ch-72C7 and GC5B596) on natural MM.1R cells expressing GPRC5D in an ADCC activity assay.

### DETAILED DESCRIPTION

Various examples of the present application will be described hereinafter for the purpose of describing the present application. Therefore, particular modifications discussed cannot be construed as limiting on the present application. It will be obvious to those skilled in the art that various equivalents, changes and modifications can be made without departing from the scope of the present application, and it should be understood that these equivalent embodiments will be included herein. All references, including the publications, patents, and patent applications, cited herein are hereby incorporated by reference in their entirety.

### Definitions

As used herein, the term "antibody" includes any immunoglobulin, monoclonal antibody, polyclonal antibody, multivalent antibody, divalent antibody, monovalent antibody, multispecific antibody or bispecific antibody that binds to a specific antigen. Natural intact antibodies include two heavy (H) chains and two light (L) chains. Mammalian heavy chains are classified into α, δ, ε, γ and µ chains, and each heavy chain consists of a variable region (V_{H}) and a first, a second, a third, and optionally a fourth constant region (C_{H1}, C_{H2}, C_{H3}, and C_{H4} respectively). Mammalian light chains are classified into λ or κ chain, and each light chain consists of a variable region (V_{L}) and a constant region. The antibody is Y-shaped, in which the stem of Y consists of the second and third constant regions of two heavy chains which are bound together by a disulfide bond. Each arm of Y contains the variable region and the first constant region of a single heavy chain binding to the variable region and the constant region of a single light chain. The variable regions of the light chain and the heavy chain are responsible for antigen binding. The variable regions of the two chains generally contain three highly variable loops, which are called complementarity determining regions (CDRs) (the light-chain CDR comprises LCDR1, LCDR2 and LCDR3, and the heavy chain CDR comprises HCDR1, HCDR2, and HCDR3). CDR boundaries of antibodies and antigen-binding fragments disclosed herein can be defined or identified by Kabat, IMGT, Chothia or Al-Lazikani (Al-Lazikani, B., Chothia, C., Lesk, A. M., J. Mol. Biol., 273(4), 927 (1997); Chothia, C. et al., J. Mol. Biol., Dec. 5;186(3):651-63 (1985); Chothia, C. and Lesk, A.M., J. Mol. Biol., 196,901 (1987); Chothia, C. et al., Nature. Dec. 21-28; 342(6252): 877-83 (1989); Kabat E.A. et al., Sequences of Proteins of immunological Interest, Fifth Edition, Public Health Service, National Institutes of Health, Bethesda, Md.) (1991); Marie-Paule Lefranc et al., Developmental and Comparative Immunology, 27: 55-77 (2003); Marie-Paule Lefranc et al., Immunome Research, 1(3), (2005); Marie-Paule Lefranc, Molecular Biology of B cells (second edition), Chapter 26, 481-514, (2015)). The three CDRs are inserted between flanking segments called framework regions (FRs), which are more conservative than CDRs and form a scaffold to support the hypervariable loops. The constant regions of the heavy chain and the light chain are not involved in antigen binding, but show various effector functions. The antibody is classified based on the amino acid sequence of the heavy-chain constant region of the antibody. The five main types or isoforms of antibodies are IgA, IgD, IgE, IgG and IgM, which are characterized by the existence of α, δ, ε, γ and µ heavy chain respectively. Several major antibody categories are classified into subclasses, such as IgG1 (γ1 heavy chain), IgG2 (γ2 heavy chain), IgG3 (γ3 heavy chain), IgG4 (γ4 heavy chain), IgA1 (α1 heavy chain) or IgA2 (α2 heavy chain).

As used herein, the term "antigen binding fragment" refers to an antibody fragment formed by a part of an antibody that includes one or more CDRs, or any other antibody fragment that binds to an antigen, but does not include an intact natural antibody structure. Examples of the antigen binding fragment include, but are not limited to, a diabody, Fab, Fab', F(ab')₂, an Fv fragment, a disulfide stabilized Fv fragment (dsFv), (dsFv)₂, a bispecific dsFv(dsFv-dsFv'), a disulfide stabilized diabody (ds diabody), a single-chain antibody molecule (scFv), an scFv dimer (a divalent diabody), a bispecific antibody, a multi-specific antibody, a camelized single-domain antibody, a nanobody, a domain antibody, and a divalent domain antibody. The antigen binding fragment can bind to the same antigen that the parent antibody binds.

The "Fab" related to an antibody refers to a part of the antibody consisting of a single light chain (variable region and constant region) bound to the variable region and the first constant region of a single heavy chain by a disulfide bond.

The Fab' refers to a Fab fragment containing a part of the hinge region.

"F(ab')₂" refers to a dimer of Fab'. The Fv of an antibody refers to the smallest antibody fragment with a complete antigen binding site. The Fv fragment consists of the variable region of a single light chain bound to the variable region of a single heavy chain.

The "dsFv" refers to a disulfide stabilized Fv fragment, in which the linkage between the variable region of a single light chain and the variable region of a single heavy chain is a disulfide bond. In some embodiments, "(dsFv)₂" or "(dsFv-dsFv')" includes three peptide chains, that is, two V_{H} moieties linked by a peptide linker (such as a long flexible linker) and bound to two V_{L} moieties, respectively, by disulfide bridges. In some embodiments, dsfv-dsfv' is bispecific, in which each pair of heavy chain and light chain linked by the disulfide bonds has a different antigen specificity.

The "single-chain Fv antibody" or "scFv" refers to an engineered antibody consisting of a light chain variable region and a heavy chain variable region linked to each other directly or by a peptide linker sequence (Huston JS et al., Proc Natl Acad Sci USA, 85:5879(1988)).

The "Fc" of an antibody (such as IgG, IgA or IgD isotype antibody) refers to a part of the antibody consisting of the second and third constant domains of a first heavy chain bound to the second and third constant domains of the second heavy chain by a disulfide bond. The Fc of IgM and IgE isotype antibodies further includes a fourth constant domain. The Fc part of an antibody is responsible for various effector functions, such as antibody-dependent cell-mediated cytotoxicity (ADCC) and complement-dependent cytotoxicity (CDC), but it does not play a role in antigen binding.

The "single chain Fv-Fc antibody" or "scFv-Fc" refers to an engineered antibody consisting of ScFv linked to the FC region of an antibody.

The "diabody" or "dAb" comprises a small antibody fragment with two antigen binding sites, where the fragment comprises a V_{H} domain linked to a V_{L} domain in the same polypeptide chain (V_{H}-V_{L} or V_{L}-V_{H}) (see, for example, Holliger P. et al., Proc Natl Acad Sci USA, July 15; 90(14): 6444-8 (1993); EP404097; WO93/11161).By using a linker that is too short to cause two domains on the same chain to pair, the domain is forced to pair with a complementary domain on the other chain, thus producing two antigen binding sites. The antigen binding sites can target the same or different antigens (or epitopes). In some embodiments, the "bispecific ds diabody" is a diabody targeting two different antigens (or epitopes). In some embodiments, the "scFv dimer" is a divalent diabody or divalent scFv (BsFv), which includes a dimer of V_{H}-V_{L} (linked by a peptide linker) with another V_{H}-V_{L} moiety, so that V_{H} in one moiety coordinates with V_{L} in another moiety to form two binding sites that can target the same antigen (or epitope) or different antigens (or epitopes). In other embodiments, the "scFv dimer" is a bispecific diabody, which includes a combination of V_{H1}-V_{L2} (linked by a peptide linker) with V_{L1}-V_{H2} (linked by a peptide linker), such that V_{H1} coordinates with V_{L1} and V_{H2} coordinates with V_{L2}, where each coordinated pair has different antigen specificity.

The "domain antibody" refers to an antibody fragment containing only the variable region of a heavy chain or the variable region of a light chain. In some cases, two or more V_{H} domains are covalently bound by a peptide linker, to produce a bivalent or multivalent domain antibody. The two V_{H} domains of a divalent domain antibody can target the same or different antigens.

As used herein, the term "chimeric" refers to an antibody or an antigen binding fragment in which a part of the heavy chain/or light chain is derived from one species and the rest of the heavy chain/or light chain is derived from a different species. In an illustrative example, the chimeric antibody may include a constant region derived from human and a variable region derived from a non-human animal such as mice. In some embodiments, the non-human animal is a mammal, such as mice, rats, rabbits, goats, sheep, guinea pigs or hamsters.

As used herein, the term "humanized" means that an antibody or an antigen binding fragment includes a CDR derived from a nonhuman animal, a FR region derived from human, and, where applicable, a constant region derived from human.

As used herein, the "bispecific" antibody refers to an artificial antibody that has fragments derived from two different monoclonal antibodies and can bind to two different epitopes. The two epitopes can exist on the same antigen, or on two different antigens.

As used herein, the "multispecific" antibody refers to an artificial antibody that has fragments derived from more than two different monoclonal antibodies and can bind to more than two different epitopes. The more than two different epitopes may exist on the same antigen or on different antigens.

As used herein, "GPRC5D" refers to G protein-coupled receptor class C group 5 member D derived from primates (such as humans and monkeys). In some embodiments, GPRC5D is human GPRC5D. An exemplary sequence of human GPRC5D includes human GPRC5D protein (UniProt No. Q9NZD1). GPRC5D is a relatively new target for immunotherapy of multiple myeloma. It is an orphan G protein-coupled receptor with unknown functions, which is highly expressed in malignant bone marrow plasma cells and hard keratinized structures including hair shaft, nails and the central area of tongue (see Smith EL et al., Sci Transl Med 2019; 11: eaau7746; Pillarisetti K et al., Blood 2020;135:1232-43.; and Inoue S et al., J Invest Dermatol 2004;122:565-73). The high expression of GPRC5D is related to the poor prognosis of multiple myeloma (see Atamaniuk J et al., Eur J Clin Invest 2012;42:953-60.). GPRC5D is used as a target of CAR-T in the treatment of multiple myeloma, with promising results achieved in preclinical studies (see de Larrea CF et al., Blood Cancer Discov 2020;1:146.); and is now a target of the bispecific antibody JNJ-64407564 (talquetamab) in four phase I trials.

The term "anti-GPRC5D antibody" refers to an antibody that can specifically bind to GPRC5D (for example, human GPRC5D). The term "anti-human GPRC5D antibody" refers to an antibody that can specifically bind to human GPRC5D.

As used herein, the term "specific binding/specifically binding" refers to a non-random binding reaction between two molecules, such as, for example, between an antibody and an antigen. The specific binding can be characterized by the binding affinity, for example, expressed by K_{D}, the ratio (k_{off}/kₒₙ) of the dissociation rate to the binding rate when the binding between an antigen and an antigen binding molecule reaches equilibrium. K_{D} can be determined by any conventional method known in the art, including, but not limited to, surface plasmon resonance, microscale thermophoresis, HPLC-MS and flow cytometry (such as FACS). An K_{D} of ≤10⁻⁶ M (for example, ≤5 x 10⁻⁷ M, ≤2 x 10⁻⁷ M, ≤10⁻⁷ M, ≤5 x 10⁻⁸ M, ≤2 x 10⁻⁸ M, ≤10⁻⁸ M, ≤5 x 10⁻⁹ M, ≤4 x 10⁻⁹M, ≤3 x 10⁻⁹M, ≤3 x 1⁻⁹ M or ≤10⁻⁹ M) indicates the specific binding between an antibody or an antigen binding fragment thereof and GPRC5D (for example, human GPRC5D).

The conservative substitution of an amino acid sequence refers to the replacement of an amino acid residue by a different amino acid residue with a side chain having similar physiological and chemical characteristics. For example, conservative substitution can be made among amino acid residues with a hydrophobic side chain (for example, Met, Ala, Val, Leu and Ile), amino acid residues with a neutral or hydrophilic side chain (for example, Cys, Ser, Thr, Asn and Gln), amino acid residues with an acidic side chain (for example, Asp, and Glu), amino acid residues with a basic side chain (for example, His, Lys, and Arg), or amino acid residues with an aromatic side chain (for example, Trp, Tyr and Phe). As is known in the art, conservative substitution usually does not cause significant changes in the conformation structure of a protein, and thus the biological activity of the protein can be retained.

As used herein, the term "homologous" means that a nucleic acid sequence (or a complementary strand thereof) or an amino acid sequence has at least 60% *(e.g.,* at least 65%, 70%, 75%, 80%, 85%, 88%, 90%, 91%, 92%, 93%, 94%, 95%, 96%, 97%, 98%, or 99%) sequence identity to another sequence in optimal alignment.

The "percent sequence identity (%)" with reference to an amino acid sequence (or a nucleic acid sequence) is defined as the percent amino acid (or nucleic acid) residues in a candidate sequence that are identical those in a reference sequence after aligning the candidate sequence with the reference sequence and introducing gaps when necessary to maximize the number of identical amino acids (or nucleic acids). Conservative substitution of amino acid residues may or may not be regarded as an identical residue. The alignment for the purpose of determining the percent amino acid (or nucleic acid) sequence identity can carried out using, for example, publicly available tools, such as BLASTN and BLASTp (available on the website of National Center for Biotechnology Information (NCBI), and see also Altschul S.F. et al., J. Mol. Biol., 215:403-410 (1990); Stephen F. et al., Nucleic Acids Res., 25:3389-3402 (1997)), ClustalW2 (available on the website of European Bioinformatics Institute, and also see Higgins D.G. et al., Methods in Enzymology, 266:383-402 (1996); Larkin M.A. et al., Bioinformatics, Oxford, England, 23(21): 2947-8 (2007)) and ALIGN or Megalign (DNASTAR) software. Those skilled in the art can use the default parameters provided by the tool, or customize the parameters suitable for alignment, such as by selecting an appropriate algorithm.

As used herein, the "effector function" refers to the biological activity caused by the binding of the Fc region of an antibody to its effector, such as the C1 complex to the Fc receptor. Exemplary effector functions include: complement-dependent cytotoxicity (CDC) mediated by the interaction of an antibody with C1q on the C1 complex; antibody-dependent cell-mediated cytotoxicity (ADCC) mediated by the binding of the Fc region of an antibody to the Fc receptor on effector cells; and phagocytosis. Various assays (such as Fc receptor binding assay, C1q binding assay and cytolysis assay) can be used to evaluate the effector function.

The "isolated" material has been changed from its natural state by artificial means. If an "isolated" composition or material exists in nature, it has been changed or removed from its original environment, or both. For example, a polynucleotide or polypeptide naturally existing in a living animal is not "isolated", but the polynucleotide or polypeptide is "isolated" if it is sufficiently separated from materials coexisting therewith in nature state and thus exists in a substantially pure state. "An isolated nucleic acid sequence" refers to the sequence of an isolated nucleic acid molecule. In some embodiments, the "isolated antibody or an antigen binding fragment thereof" means an antibody or an antigen binding fragment thereof having a purity of at least 60%, 70%, 75%, 80%, 81%, 82%, 83%, 84%, 85%, 86%, 87%, 88%, 89%, 90%, 91%, 92%, 93%, 94%, 95%, 96%, 97%, 98%, or 99% as determined by electrophoresis (such as SDS-PAGE, isoelectric focusing, and capillary electrophoresis) or chromatography (such as ion exchange chromatography or reversed-phase HPLC).

As used herein, the term "vector" refers to a vehicle into which a gene element is operably inserted to realize the expression of the gene element, thereby generating a protein, RNA or DNA encoded by the gene element, or replicating the gene element. The vector can be used to transform, transduct or transfect the host cells, so that the gene elements carried can be expressed in the host cells. Examples of vectors include plasmids; phagemids; cosmids; artificial chromosome, such as yeast artificial chromosome (YAC), bacterial artificial chromosome (BAC) or P1-derived artificial chromosome (PAC); phage, such as λ phage or M13 phage; and animal viruses. The vector can contain various elements for controlling expression, including a promoter sequence, a transcription initiation sequence, an enhancer sequence, a selection element and a reporter gene. In addition, the vector can contain the origin of replication. The vector can also contain substances promoting the entry into cells, including, but not limited to, virus particles, liposomes or protein envelopes. The vector can be an expression vector or a cloning vector. The present application provides a vector (such as an expression vector) containing a nucleic acid sequence encoding the antibody or antigen binding fragment thereof provided herein, at least one promoter (such as SV40, CMV, and EF-1α) operably linked to the nucleic acid sequence, and at least one selectable marker.

As used herein, the phrase "host cell" refers to a cell into which an exogenous polynucleotide and/or vector can be or has been introduced.

As used herein, the "treating" or "treatment" of a condition includes alleviating the condition, slowing down the onset or development rate of the condition, reducing the risk of suffering from the condition, delaying the development of symptoms related to the condition, reducing or eliminating symptoms related to the condition, causing complete or partial regression of the condition, and curing the condition or some combination thereof.

As used herein, the "GPRC5D-related" disease or condition refers to any disease or condition caused or aggravated by, or otherwise related to the increase or decrease of the expression or activity of GPRC5D. In some embodiments, the GPRC5D-related diseases or condition is cancers, such as myeloma. In some embodiments, the GPRC5D-related disease or condition is characterized by over-expression of GPRC5D gene. In one embodiment, the GPRC5D-related disease or condition includes, but is not limited to, GPRC5D positive breast cancer, multiple myeloma, Waldenstörm macroglobulinemia, endometrial cancer, ovarian cancer, lung cancer, gastric cancer, prostate cancer, renal cancer, liver cancer, pancreatic cancer, colorectal cancer, esophageal cancer, bladder cancer, cervical cancer, hematological cancer, lymphoma or malignant melanoma.

The term "pharmaceutically acceptable" indicates that the specified carrier, vehicle, diluent, excipient and/or salt are generally chemically and/or physically compatible with other ingredients constituting the formulation and physiologically compatible with its recipient.

### Anti-GPRC5D antibody,

The present application provides an anti-GPRC5D antibody or an antigen binding fragment thereof. The anti-GPRC5D antibody or antigen binding fragment thereof provided herein can specifically bind to GPRC5D.

In some embodiments, the antibody and antigen binding fragment thereof provided herein bind to human GPRC5D at a K_{D} of no more than 8 x 10⁻⁸ M, no more than 5 x 10⁻⁸ M, no more than 2 x 10⁻⁸ M, no more than 8 x 10⁻⁹ M, no more than 5 x 10⁻⁹ M, no more than 2 x 10⁻⁹ M, or no more than 10⁻⁹ M as shown by Biacore analysis. The Biacore analysis is based on the surface plasmon resonance technology, see, for example, Murphy, M. et al., Current protocols in protein science, 2006.Chapter 19, Unit 19.14, 2006.

The binding of the antibody to human GPRC5D can also be expressed by the "median effective concentration" (EC₅₀), and EC₅₀ refers to the antibody concentration when 50% of maximum binding is observed. The EC₅₀ value can be measured by binding assays known in the art, such as sandwich assays, such as enzyme-linked immunosorbent assay (ELISA), flow cytometry and other binding assays. In some embodiments, as measured by flow cytometry, the antibody and a fragment thereof provided herein specifically bind to cells expressing human GPRC5D at an EC₅₀ value (i.e. 50% binding concentration) of no more than 1 µg/ml, no more than 2 µg/ml, no more than 3 µg/ml, no more than 4 µg/ml, no more than 5 µg/ml, and no more than 10 µg/ml.

As used herein, the "binding ability" refers to the ability of a molecule (such as an antibody) to bind to another molecule (such as an antigen). The ability can be measured by, for example, the binding activity with an antigen of interest using any suitable binding assay known in the art. For example, the antibody of interest can be labeled to allow for the direct quantification of the binding activity with the antigen. For another example, the binding activity of an antibody of interest (i.e., a primary antibody) to its antigen can also be detected by using a labeled secondary antibody (e.g., an anti-species antibody) that detects, by binding to the primary antibody in the complex, the complex formed by the primary antibody bound to its antigen, and thus indirectly quantifies the binding activity. The labeled antibody can be detected by, for example, enzyme-linked immunosorbent assay (ELISA, for example, where the label is an enzyme), flow cytometry (for example, where the label is fluorescent), Western blotting (for example, the label is a fluorescent or radioactive ligand), colorimetry, and chemiluminescence-based methods, etc.

In some embodiments, the present application provides an anti-GPRC5D monoclonal antibody ch-72C7.

As used herein, "ch-72C7" refers to monoclonal antibody having a light chain variable region comprising a sequence as shown in SEQ ID NO: 12 and a heavy chain variable region comprising a sequence as shown in SEQ ID NO: 13. ch-72C7 is an antibody from mice, in which the CDR sequence can be divided by methods known in the art, including, but not limited to, IMGT, or based on Kabat numbering.

Table 1 below shows the CDR sequence of antibody ch-72C7 divided based on IMGT numbering. Table 2 below shows the CDR sequence of antibody ch-72C7 divided based on Kabat numbering. Table 3 below shows the amino acid sequences of the heavy chain and light chain variable region of GPRC5D.

**Table 1. Amino acid sequence of CDR of antibody ch-72C7 based on IMGT numbering**

| | | **CDR1** | **CDR2** | **CDR3** |
|---|---|---|---|---|
| **ch-72C7** | **LCDR** | SEQ ID NO: 1 SSVSF | SEQ ID NO: 2 DTT | SEQ ID NO: 3 QQWNSHPLT |
| | **HCDR** | SEQ ID NO: 4 GYPFTNYW | SEQ ID NO: 5 INPSNGRT | SEQ ID NO: 6 ARGFAY |

**Table 2. Amino acid sequence of CDR of antibody ch-72C7 divided based on Kabat numbering**

| | | **CDR1** | **CDR2** | **CDR3** |
|---|---|---|---|---|
| **ch-72C7** | **LCDR** | SEQ ID NO: 7 SASSSVSFMH | SEQ ID NO: 8 DTTKLAS | SEQ ID NO: 3 QQWNSHPLT |
| | **HCDR** | SEQ ID NO: 9 NYWMH | SEQ ID NO: 10 EINPSNGRTNYNEKFKS | SEQ ID NO: 11 GFAY |

**Table 3. Amino acid sequence of variable region of ch-72C7**

| | **VL** | **VH** |
|---|---|---|
| **ch-72C7** | SEQ ID NO: 12 QIVLSQFPAIMSASPGEKVTMT CSASSSVSFMHWYQQKSGTSP KRWIYDTTKLASGVPARFTGSG SGTSYSLTISSMEAEDAATYYC QQWNSHPLTFGPGTKLELK | SEQ ID NO: 13 QVQLQQPGAELVKPGTSVKLSCKAS GYPFTNYWMHWVRQRPGQGLEWI GEINPSNGRTNYNEKFKSLATLTLDK SSSTAYMHLNSLTSEDSAVYYCARG FAYWGQGTLVTVSA |

In some embodiments, the present application provides an anti-GPRC5D antibody and an antigen binding fragment thereof, which includes one or more *(e.g.* 1, 2, 3, 4, 5 or 6) CDR sequences of antibody ch-72C7.

In some embodiments, the present application provides an anti-GPRC5D antibody (for example, anti-human GPRC5D antibody) and an antigen binding fragment thereof, which includes three heavy chain complementarity determining regions HCDR1, HCDR2 and HCDR3, and three light chain complementarity determining regions LCDR1, LCDR2, and LCDR3. The three heavy chain complementarity determining regions are identical to three heavy chain complementarity determining regions comprised in a heavy chain variable region (V_{H}) as shown in SEQ ID NO: 13, and the three light chain complementarity determining regions are identical to three light chain complementarity determining regions comprised in a light chain variable region (V_{L}) as shown in SEQ ID NO: 12.

In some embodiments, the present application provides an anti-GPRC5D antibody (for example, anti-human GPRC5D antibody) and an antigen binding fragment thereof, which includes one or more *(e.g.* 1, 2, 3, 4, 5 or 6) CDRs. The one or more CDRs include a sequence selected from the group consisting of SSVSF (SEQ ID NO: 1), DTT (SEQ ID NO: 2), QQWNSHPLT (SEQ ID NO: 3), GYPFTNYW (SEQ ID NO: 4), INPSNGRT (SEQ ID NO: 5) and ARGFAY (SEQ ID NO: 6).

In some embodiments, the present application provides an anti-GPRC5D antibody (for example, anti-human GPRC5D antibody) and an antigen binding fragment thereof, which includes one or more *(e.g.* 1, 2, 3, 4, 5 or 6) CDRs. The one or more CDRs include a sequence selected from the group consisting of SASSSVSFMH (SEQ ID NO: 7), DTTKLAS (SEQ ID NO: 8), QQWNSHPLT (SEQ ID NO: 3), NYWMH (SEQ ID NO: 9), EINPSNGRTNYNEKFKS (SEQ ID NO: 10) and GFAY (SEQ ID NO: 11).

In some embodiments, the HCDR1 comprises the amino acid sequence selected from the group consisting of SEQ ID NO: 4 or a variant thereof having no more than 3, 2 or 1 amino acid substitution, or SEQ ID NO: 9 or a variant thereof having no more than 3, 2 or 1 amino acid substitution. The HCDR2 includes the amino acid sequence selected from the group consisting of SEQ ID NO: 5 or a variant thereof having no more than 3, 2 or 1 amino acid substitution, or SEQ ID NO: 10 or a variant thereof having no more than 3, 2 or 1 amino acid substitution. The HCDR3 includes the amino acid sequence selected from the group consisting of SEQ ID NO: 6 or a variant thereof having no more than 3, 2 or 1 amino acid substitution, or SEQ ID NO: 11 or a variant thereof having no more than 3, 2 or 1 amino acid substitution. The LCDR1 includes the amino acid sequence selected from the group consisting of SEQ ID NO: 1 or a variant thereof having no more than 3, 2 or 1 amino acid substitution, or SEQ ID NO: 7 or a variant thereof having no more than 3, 2 or 1 amino acid substitution. The LCDR2 includes the amino acid sequence selected from the group consisting of SEQ ID NO: 2 or a variant thereof having no more than 3, 2 or 1 amino acid substitution, or SEQ ID NO: 8 or a variant thereof having no more than 3, 2 or 1 amino acid substitution. The LCDR3 includes the amino acid sequence selected from the group consisting of SEQ ID NO: 3 or a variant thereof having no more than 3, 2 or 1 amino acid substitution.

In some embodiments, in the anti-GPRC5D antibody or antigen binding fragment thereof provided in the present application, the HCDR1 comprises the amino acid sequence as shown in SEQ ID NO: 4 or a variant thereof having no more than 3, 2 or 1 amino acid substitution, the HCDR2 comprises the amino acid sequence as shown in SEQ ID NO: 5 or a variant thereof having no more than 3, 2 or 1 amino acid substitution, the HCDR3 comprises the amino acid sequence as shown in SEQ ID NO: 6 or a variant thereof having no more than 3, 2 or 1 amino acid substitution, the LCDR1 comprises the amino acid sequence as shown in SEQ ID NO: 1 or a variant thereof having no more than 3, 2 or 1 amino acid substitution, the LCDR2 comprises the amino acid sequence as shown in SEQ ID NO: 2 or a variant thereof having no more than 3, 2 or 1 amino acid substitution, and the LCDR3 comprises the amino acid sequence as shown in SEQ ID NO: 3 or a variant thereof having no more than 3, 2 or 1 amino acid substitution.

In some embodiments, in the anti-GPRC5D antibody or antigen binding fragment thereof provided in the present application, the HCDR1 comprises the amino acid sequence as shown in SEQ ID NO: 9 or a variant thereof having no more than 3, 2 or 1 amino acid substitution, the HCDR2 comprises the amino acid sequence as shown in SEQ ID NO: 10 or a variant thereof having no more than 3, 2 or 1 amino acid substitution, the HCDR3 comprises the amino acid sequence as shown in SEQ ID NO: 11 or a variant thereof having no more than 3, 2 or 1 amino acid substitution, the LCDR1 comprises the amino acid sequence as shown in SEQ ID NO: 7 or a variant thereof having no more than 3, 2 or 1 amino acid substitution, the LCDR2 comprises the amino acid sequence as shown in SEQ ID NO: 8 or a variant thereof having no more than 3, 2 or 1 amino acid substitution, and the LCDR3 comprises the amino acid sequence as shown in SEQ ID NO: 3 or a variant thereof having no more than 3, 2 or 1 amino acid substitution.

In some embodiments, the amino acid substitutions are conservative substitutions. In some embodiments, the amino acid substitution does not occur in the CDR region. In some embodiments, the variant still retains the specific binding affinity to GPRC5D (such as human GPRC5D).

In some embodiments, the anti-GPRC5D antibody or antigen binding fragment thereof provided in present application comprises LCDR1 comprising the amino acid sequence as shown in SEQ ID NO: 1, LCDR2 comprising the amino acid sequence as shown in SEQ ID NO: 2, and LCDR3 comprising the amino acid sequence as shown in SEQ ID NO: 3, and/or HCDR1 comprising the amino acid sequence as shown in SEQ ID NO: 4, HCDR2 comprising the amino acid sequence as shown in SEQ ID NO: 5, and HCDR3 comprising the amino acid sequence as shown in SEQ ID NO: 6.

In some embodiments, the anti-GPRC5D antibody or antigen binding fragment thereof provided in present application comprises LCDR1 comprising the amino acid sequence as shown in SEQ ID NO: 7, LCDR2 comprising the amino acid sequence as shown in SEQ ID NO: 8, and LCDR3 comprising the amino acid sequence as shown in SEQ ID NO: 3, and/or HCDR1 comprising the amino acid sequence as shown in SEQ ID NO: 9, HCDR2 comprising the amino acid sequence as shown in SEQ ID NO: 10, and HCDR3 comprising the amino acid sequence as shown in SEQ ID NO: 11.

CDR is known to be responsible for antigen binding. However, it has been found that not all 6 CDRs are essential or unchangeable. In other words, it is possible to replace, change or modify one or more CDRs in the anti-GPRC5D antibody ch-72C7, with the specific binding affinity for GPRC5D being substantially retained.

In some embodiments, the anti-GPRC5D antibody or antigen binding fragment thereof provided herein includes a heavy chain CDR3 sequence of antibody ch-72C7. In some embodiments, the anti-GPRC5D antibody or antigen binding fragment thereof provided herein comprises a heavy chain CDR3 sequence as shown in SEQ ID NO: 6, where the CDR3 is numbered based on IMGT numbering. In some embodiments, the anti-GPRC5D antibody or antigen binding fragment thereof provided herein comprises a heavy chain CDR3 sequence as shown in SEQ ID NO: 11, where the CDR3 is numbered based on Kabat numbering.

In some embodiments, the antibody or antigen binding fragment thereof provided herein includes any suitable framework region (FR) sequence, as long as the antibody and antigen binding fragment thereof can specifically bind to GPRC5D. The CDR sequence provided in Table 1 or Table 2 above is obtained from a mouse antibody, but it can be transplanted into any suitable FR sequence of any suitable species such as mouse, human, rat, and rabbit, etc. using suitable methods known in the art, such as recombinant technology.

In some embodiments, the anti-GPRC5D antibody or antigen binding fragment thereof provided herein is humanized. The humanized antibody or antigen-binding fragment is desirable in reducing the immunogenicity in human. The humanized antibody is chimeric in its variable region, because the non-human CDR sequence is transplanted into a human or substantially human FR sequence. The humanization of an antibody or an antigen-binding fragment can basically be carried out by replacing a non-human (such as mouse) CDR gene with a corresponding human CDR gene in human immunoglobulin gene (see, for example, Jones et al. (1986) Nature, 321:522-525; Riechmann et al. (1988) Nature, 332:323-327; Verhoeyen et al. (1988) Science 239:1534-1536).

Methods known in the art can be used to select suitable human heavy chain and light chain variable domains for this purpose. In an illustrative example, the "best fit" method can be used, in which a variable domain sequence of a non-human (e.g., rodent) antibody is screened or blasted against a database of known human variable domain sequences, and a human sequence closest to a non-human query sequence is identified, and used as a human scaffold for transplanting a non-human CDR sequence (see, for example, Sims et al., (1993) J Immunol. 151: 2296; and Chothia et al. (1987) J. Mol. Biol. 196: 901). Alternatively, the framework derived from the consensus sequence of all human antibodies can be used for the transplantation of non-human CDRs (see, for example, Carter et al. (1992) Proc Natl Acad Sci USA, 89:4285; and Presta et al. (1993) J Immunol.,151:2623).

In some embodiments, the humanized antibody or antigen binding fragment thereof provided herein consists essentially of human sequences except that the CDR sequence is non-human. In some embodiments, the variable region FR and the constant region, if present, are completely or substantially derived from the human immunoglobulin sequence. The human FR sequence and the human constant region sequence can be derived from different human immunoglobulin genes. For example, the FR sequence is derived from one human antibody and the constant region is derived from another human antibody. In some embodiments, the humanized antibody or antigen-binding fragment comprises human heavy chain HFR1-4 and/or light chain LFR1-4.

In some embodiments, the human-derived FR region may include the same amino acid sequence as the human immunoglobulin from which it is derived. In some embodiments, one or more amino acid residues of human FR are substituted with a corresponding residue from a parent non-human antibody. In some embodiments, it may be necessary to closely approximate the structure of a humanized antibody or a fragment thereof to a non-human parent antibody. In some embodiments, the humanized antibody or antigen binding fragment provided herein includes no more than 10, 9, 8, 7, 6, 5, 4, 3, 2 or 1 amino acid residue substitution in each human FR sequence, or no more than 10, 9, 8, 7, 6, 5, 4, 3, 2 or 1 amino acid residue substitution in all FRs of the heavy chain or light chain variable domain. In some embodiments, such changes in amino acid residues can exist only in the FR region of the heavy chain, only in the FR region of the light chain, or in both chains.

In some embodiments, one or more amino acid residues are mutated, for example, back to the corresponding residues found in a non-human parent antibody from which the CDR sequence is derived (for example, in the mouse framework region). Those skilled in the art can select the appropriate mutation position according to the principles known in the art. For example, a position for mutation can be selected, in which: 1) there are few residues in the framework of the human phylogenetic sequence (for example, less than 20% or less than 10% in the human variable region sequence); 2) because this position may interact with a residue in CDR, it is immediately adjacent to one or more of the 3 CDRs in the primary sequence of the human phylogenetic chain; or 3) the position is close to CDR in the 3D model, so the possibility of interaction with an amino acid in CDR is high. The residue at the selected position can be mutated back to the corresponding residue in the parent antibody, or mutated into a corresponding in neither the human phylogenetic sequence nor the parent sequence, but a typical residue in human sequence. That is, in a known human sequence belonging to the same subgroup with the human phylogenetic sequence, it occurs more frequently at this position (see US Patent No. 5,693,762).

In some embodiments, the humanized light chain and heavy chain described in the present application are basically non-immunogenic in humans, and retain basically the same or even higher affinity compared with the parent antibody against GPRC5D.

The present application also provides an exemplary humanized antibody of ch-72C7, including:
1) "22Mono5JO4", comprising a heavy chain variable region (22Mono5JO4-VH) with an amino acid sequence as shown in SEQ ID NO: 15 and a light chain variable region (22Mono5JO4-VL) with an amino acid sequence as shown in SEQ ID NO: 14;
2) "22Mono3L7F", comprising a heavy chain variable region (22Mono3L7F-VH) with an amino acid sequence as shown in SEQ ID NO: 17 and a light chain variable region (22Mono3L7F-VL) with an amino acid sequence as shown in SEQ ID NO: 16;
3) "22Mono4DN4", comprising a heavy chain variable region (22Mono4DN4-VH) with an amino acid sequence as shown in SEQ ID NO: 19 and a light chain variable region (22Mono4DN4-VL) with an amino acid sequence as shown in SEQ ID NO: 18; and
4) "22Mono5UQY", comprising a heavy chain variable region (22Mono5UQY-VH) with an amino acid sequence as shown in SEQ ID NO: 21 and a light chain variable region (22Mono5UQY-VL) with an amino acid sequence as shown in SEQ ID NO: 20.

**Table 4. Amino acid sequence of variable region of humanized anti-GPRC5D antibody**

| | VL | VH |
|---|---|---|
| 22Mono5JO4 | SEQ ID NO: 14 EIVLTQSPGTLSLSPGERATLSC SASSSVSFMHWYQQKPGQAPR RWIYDTTKLASGIPDRFSGSGS GTDYTLTISRLEPEDFAVYYCQ QWNSHPLTFGQGTTVEIK | SEQ ID NO: 15 QVQLVQSGAEVKKPGSSVKVS CRASGYPFTNYWMHWVRQAP GQGLEWIGEINPSNGRTNYNEK FKSRATLTLDKLTSTAYMELSSL TSEDTALYYCARGFAYWGQGT LVTVSS |
| 22Mono3L7F | SEQ ID NO: 16 EIVLTQSPATLSLSPGERATLSCS ASSSVSFMHWYQQKPGQAPRR WIYDTTKLASGIPARFSGSGSG TDYTLTISSLEPEDFAVYYCQQ WNSHPLTFGQGTKLEIK | SEQ ID NO: 17 EVTLKESGPVLVKPTETLTLTCT ASGYPFTNYWMHWVRQPPGK ALEWIGEINPSNGRTNYNEKFK SRATLSLDKSKSQAVLTMTNM DPVDTATYYCARGFAYWGQGT LVTVSS |
| 22Mono4DN4 | SEQ ID NO: 18 EIVLTQSPATLSLSPGERATLSCS ASSSVSFMHWYQQKPGQAPRR WIYDTTKLASGVPARFSGSGSG TDYTLTISSLEPEDFAVYYCQQ WNSHPLTFGQGTKVEIK | SEQ ID NO: 19 ELVQSGAEVKKPGSSVKVSCK ASGYPFTNYWMHWVRQAPGQ GLEWIGEINPSNGRTNYNEKFK SRATLTLDKSTSTAYMELSSLRS EDTAVYYCARGFAYWGQGTLV TVSS |
| 22Mono5UQY | SEQ ID NO: 20 AIQLTQSPSSLSASVGDRVTITC SASSSVSFMHWYQQKPGKAPK RWIYDTTKLASGVPSRFSGSGS GTDYTFTISSLQPEDIATYYCQQ WNSHPLTFGPGTKVDIK | SEQ ID NO: 21 QVQLQESGPGLVKPSETLSLTC TASGYPFTNYWMHWVRQPPG KGLEWIGEINPSNGRTNYNEKF KSRATLSLDKSKNQASLNLDSV SAADTAIYYCARGFAYWGKGS TVTVSS |

In some embodiments, the present application further provides a humanized anti-GPRC5D antibody or an antigen binding fragment thereof, which comprises a HFR1, HFR2, HFR3 and/or HFR4 sequence in a heavy chain variable region. The heavy chain variable region is selected from the group consisting of 22Mono5JO4-VH (SEQ ID NO: 15), 22Mono3L7F-VH (SEQ ID NO: 17), 22Mono4DN4-VH (SEQ ID NO: 19) and 22Mono5UQY-VH (SEQ ID NO: 21).

In some embodiments, the present invention further provides a humanized anti-GPRC5D antibody or an antigen binding fragment thereof, which comprises a LFR1, LFR2, LFR3 and/or LFR4 sequence in a light chain variable region. The light chain variable region is selected from the group consisting of 22Mono5JO4-VL (SEQ ID NO: 14),

ASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKV (SEQ ID NO:
24)

In some embodiments, the CL region in the anti-GPRC5D antibody or antigen binding fragment thereof of the present application comprises a sequence as shown in SEQ ID NO: 25.



 (SEQ ID NO: 25)

In some embodiments, the heavy chain constant region includes a Fc region. The Fc region is known to mediate the effector functions, such as ADCC and CDC of the antibody. The Fc region of different Ig isotypes has different ability to induce the effector function. For example, it has been recognized that the Fc regions of IgG1 and IgG3 induce ADCC and CDC more effectively than the Fc regions of IgG2 and IgG4. In some embodiments, the anti-GPRC5D antibody and fragment thereof provided herein further comprise the constant region of human IgG1, IgG2, IgG3 or IgG4. In some embodiments, the anti-GPRC5D antibody and antigen binding fragment thereof provided herein include the Fc region of IgG1 or IgG3 isotype, which can induce ADCC or CDC; or, the constant region of IgG4 or IgG2 isotype, which has the effector function of reduction or depletion.

An antigen binding fragment against GPRC5D is also provided. Various types of antigen binding fragments are known in the art and can be developed based on the anti-GPRC5D antibody provided herein, including, for example, exemplary antibodies with CDR sequences and variable sequences shown in Tables 1 and 2 and their various variants (such as affinity variants, glycosylation variants, Fc variants, and cysteine engineered variants, etc., as described in a later section in the present application).

In some embodiments, the antigen binding fragment against GPRC5D provided herein is a diabody, a single-chain Fv fragment (scFv), a scFv dimer, BsFv, a disulfide-stabilized Fv (dsFv), (dsFv)₂, dsFv-dsFv', a Fv fragment, Fab, Fab', F(ab')₂, a bispecific antibody, a ds diabody, a domain antibody, a single-domain antibody, or a divalent domain antibody.

Various techniques can be used to make such antigen binding fragments. Exemplary methods include enzymatic digestion of intact antibodies (see, for example, Morimoto et al., Journal of Biochemical and Biophysical Methods, 24:107-117 (1992); and Brennan et al., Science, 229:81 (1985)), recombinant expression by host cells *(e.g.* E. Coli) (for example, for antibody fragments Fab, Fv and ScFv), screening from a phage display library (for example, for ScFv) as discussed above and chemically coupling of two Fab'-SH fragments to form a F(ab')₂ fragment (Carter et al., Bio/Technology 10: 163-167 (1992)).Other techniques for producing antibody fragments will be apparent to the skilled person.

In some embodiments, the antigen binding fragment against GPRC5D provided in the present application is a disulfide-stabilized Fv. The crystal structure analysis of the antibody shows that cysteine mutations can be introduced into some relatively conservative sequences at the VL-VH interface, thus forming a disulfide bond between VL and VH, which makes them covalently linked. The covalent bond between VL and VH significantly improves the stability of the antibody. The original dsFv (disulfide stabilized Fv) is constructed by introducing a disulfide bond at the VH-VL interface through the covalent interaction between cysteine residues in the CDR of each fragment (see Glockshuber, R. et al., A comparison of strategies to stabilize immunoglobulin Fv-fragments, (1990) Biochemistry, 291362-1367). Although the activity of an antibody will not be affected by this method, the "customized" design needs the detailed structural information of the CDR of the original antibody to avoid the interference with the antigen recognition/binding ability of the CDR. This makes this method difficult to be a general solution for constructing various antibodies. To ensure the wide use of this method, amino acids at selected sites in conserved FR is required to participate in the construction of dsFv.

Since 1993, several pairs of sites suitable for forming covalent bonds between VH-VL have been found, including VH44-VL100, VH105-VL43, VH100b-VL49, VH100-VL150 and VH101-VL46, etc., wherein the numbering is based on Kabat numbering (see Reiter, Y. et al., Stabilization of the Fv fragments in recombinant immunotoxins by disulfide bonds engineered into conserved framework regions, (1994) Biochemistry, 335451-5459; Jung,S.H. et al., Design of interchain disulfide bond in the framework region of Fv fragment of monoclonal antibody B3, (1994) Protein, Structure, Function, and Gene, 19, 35-47; Glockshuber, R. et al., A comparison of strategies to stabilize immunoglobulin Fv-fragments, (1990) Biochemistry, 291362-1367; and Zhu, Z. et al., Remodeling domain interfaces to enhance heterodimer formation, (1997) Protein Science, 6, 781-788) . VH44-VL100 and VH105-VL43 are better than other sites in many aspects (such as protein expression level, purity, Tm and affinity) to some degrees, so they are widely used.

In some embodiments, the antigen binding fragment against GPRC5D provided in the present application is scFv. The production of scFv is described in, for example, WO 93/16185; and US Patent Nos. 5,571,894 and 5,587,458. The scFv can be fused with an effector protein at the amino or carboxyl terminus to provide a fusion protein (see, for example, Antibody Engineering, Borrebaek eds).

In some embodiments, the antibody and fragment thereof provided herein have specific binding affinity for human GPRC5D, which is sufficient to provide the diagnostic and/or therapeutic use.

### Antibody variants

The antibody or antigen binding fragment thereof provided herein also cover various antibody variants thereof.

In some embodiments, the antibody variants include one or more modifications or substitutions in one or more CDR sequences provided in Tables 1 and 2 above, one or more variable region sequences provided in Tables 3 and 4 above (but not in any CDR sequence) and/or constant regions (such as Fc regions). Such variants retain the binding specificity of their parent antibody to the antigen GPRC5D, and have one or more desired characteristics endowed by one or more modifications or one or more substitutions. For example, the antibody variants may have increased antigen binding affinity, improved glycosylation pattern, reduced glycosylation risk, reduced deamination, reduced or removed effector function(s), improved FcRn receptor binding, increased pharmacokinetic half-life, pH sensitivity and/or compatibility with binding (e.g., one or more introduced cysteine residues).

Methods known in the art, such as "alanine scanning mutagenesis" (see, for example, Cunningham and Wells (1989) Science, 244:1081-1085), can be used to screen the parent antibody sequence to identify suitable or preferred residues for modification or substitution. Briefly, target residues (for example, charged residues such as Arg, Asp, His, Lys and Glu) can be recognized, and the target residues can be replaced by neutral or negatively charged amino acids (for example, alanine or polyalanine), to produce modified antibodies that are screened for characteristics of interest. If a substitution at a specific amino acid position shows a functional change of interest, the position can be identified as a potential residue for modification or substitution. The potential residues can be further evaluated by substitution with different types of residues (e.g. cysteine residues, and positively charged residues, etc.).

### Affinity variants

The affinity variants may contain modifications or substitutions in one or more CDR sequences provided in Table 1 or 2 above, one or more FR sequences provided herein, or a heavy chain or light chain variable region sequence provided in Table 3 or 4 above. The FR sequences can be easily identified by those skilled in the art based on the CDR sequences in Table 1 or 2 and the variable region sequences in Table 3 or 4 above, because it is well known in the art that the CDR region is flanked by two FR regions in the variable region. The affinity variants retain the specific binding affinity of the parent antibody to the antigen GPRC5D, or even have an improved specific binding affinity to the antigen GPRC5D superior to that of the parent antibody. In some embodiments, at least one (or all) substitution in the CDR sequence, FR sequence or variable region sequence includes a conservative substitution.

It should be understood by those skilled in the art that in the CDR sequences and variable region sequences provided in Tables 1-4 above, one or more amino acid residues can be substituted, and the resulting antibody or antigen binding fragment still retains the binding affinity or binding ability to the antigen GPRC5D, or even has an improved binding affinity or ability. Various methods known in the art can be used to achieve this goal. For example, a library of antibody variants (such as Fab or scFv variants) can be produced and expressed by the phage display technology, and then screened for the binding affinity to the corresponding antigen GPRC5D. For another example, computer software can be used to practically simulate the binding of an antibody to the antigen GPRC5D, and to identify amino acid residues on the antibody forming a binding interface. Substitution of such residues can be avoided to prevent the decrease of the binding affinity, or such residues can be used as the target of substitution to achieve stronger binding.

In some embodiments, the antibody or antigen binding fragment provided herein include one or more substitutions of amino acid residues in one or more CDR sequences and/or one or more FR sequences. In some embodiments, the affinity variants include not more than 20, 15, 10, 9, 8, 7, 6, 5, 4, 3, 2 or 1 substitution in total in the CDR sequence and/or FR sequence.

In some embodiments, the anti-GPRC5D antibody or antigen binding fragment thereof comprises 1, 2 or 3 CDR sequences having at least 80% *(e.g.,* at least 85%, 88%, 90%, 91%, 92%, 93%, 94%, 95%, 96%, 97%, 98% or 99%) sequence identity to (those) sequences listed in Table 1 or Table 2 above, and retains a GPRC5D binding affinity comparable to or even higher than that of the parent antibody.

In some embodiments, the anti-GPRC5D antibody or antigen binding fragment thereof comprises one or more variable region sequences having at least 80% *(e.g.,* at least 85%, 88%, 90%, 91%, 92%, 93%, 94%, 95%, 96%, 97%, 98% or 99%) sequence identity to (those) sequences listed in Table 3 or 4 above, and retains a GPRC5D binding affinity comparable to or even higher than that of the parent antibody. In some embodiments, the variable region sequence listed in Table 3 above has 1 to 10 amino acid substitutions, insertions or deletions in total. In some embodiments, the substitution, insertion or deletion occurs in regions outside the CDR *(e.g.,* in FR).

### Glycosylation variants

The anti-GPRC5D antibody or antigen binding fragment thereof provided herein also covers glycosylation variants, which can be obtained to increase or decrease the extent of glycosylation of the antibodies or antigen-binding fragments.

The antibody or antigen binding fragment thereof may include one or more modifications to introduce or remove the glycosylation sites. The glycosylation site is an amino acid residue having a side chain that can be linked to a carbohydrate moiety (such as a oligosaccharide structure). Glycosylation of the antibody is usually N-linked or O-linked. N-linked refers to the linkage of a carbohydrate moiety to the side chain of an asparagine residue (for example, the asparagine residue in a tripeptide sequence such as asparagine-X-serine and asparagine-X-threonine, where X is any amino acid except proline). O-linked glycosylation refers to the linkage of one of N-acetylgalactosamine, galactose or xylose to a hydroxyamino acid, most commonly to serine or threonine. The removal of a natural glycosylation site can be conveniently achieved, for example, by changing the amino acid sequence so that one of the tripeptide sequences described above (for N-linked glycosylation site) or the serine or threonine residue present in the sequence (for O-linked glycosylation site) is replaced. In a similar manner, a new glycosylation site can be produced by introducing such a tripeptide sequence or a serine or threonine residue.

In some embodiments, the anti-GPRC5D antibody or antigen binding fragment thereof provided herein include mutations at N297 (e.g., N297A, N297Q or N297G) to remove the glycosylation site.

### Cysteine engineered variants

The anti-GPRC5D antibody or antigen binding fragment thereof provided herein also cover a cysteine engineered variant, which includes one or more introduced free cysteine residues.

The free cysteine residue is a cysteine residue that is not part of a disulfide bridge. The cysteine engineered variant can be bound to for example, a cytotoxic and/or imaging compound, a label, or a radioisotope at the engineered cysteine site, for example, by a maleimide or haloacetyl radical. Methods for engineering an antibody or an antigen-binding fragment thereof to introduce a free cysteine residue are known in the art, see, for example, WO2006/034488.

### Fc variants

The anti-GPRC5D antibody or antigen binding fragment thereof provided herein also covers an Fc variant, which include one or more modifications or substitutions of amino acid residues in the Fc region and/or hinge region, for example, to provide a changed effector function, such as ADCC and CDC. Methods of changing the ADCC activity by antibody engineering are described in the art, see, for example, Shields RL. et al., J Biol Chem. 2001. 276(9): 6591-604; Idusogie EE. et al., J Immunol. 2000.164(8):4178-84; Steurer W. et al., J Immunol. 1995, 155(3): 1165- 74; Idusogie EE. et al., J Immunol. 2001, 166(4): 2571-5; Lazar GA. et al., Proc. Natl. Acad. USA, 2006, 103(11): 4005-4010; and Ryan MC. et al., Mol. Cancer Ther., 2007, 6: 3009-3018; Richards JO,. et al., Mol. Cancer Ther. 2008, 7(8): 2517-27; Shields R. L. et al., J. Biol. Chem, 2002, 277: 26733-26740; Shinkawa T. et al., J. Biol. Chem, 2003, 278: 3466-3473.

The CDC activity of the antibody provided herein can also be changed, for example, by improving or weakening the C1q binding and/or CDC (see, for example, WO99/51642; Duncan and Winter, Nature 322: 738-40 (1988); US Patent No. 5,648,260; US Patent No. 5,624,821); and for other examples of Fc region variants, see WO94/29351. One or more amino acids selected from amino acid residues 329, 331 and 322 in the Fc region can be substituted with different amino acid residues to change the Clq binding and/or reduce or eliminate the complement-dependent cytotoxicity (CDC) (see US Patent No. 6,194,551 to Idusogie et al.). One or more amino acid substitutions can also be introduced to change the antibody's ability to fix the complement (see PCT Publication No. WO 94/29351 to Bodmer et al.).

In some embodiments, the anti-GPRC5D antibody or antigen binding fragment thereof provided herein has a reduced effector function and include one or more amino acid substitutions in IgG1 at positions selected from the group consisting of 234, 235, 237 and 238, 268, 297, 309, 330 and 331. In some embodiments, the anti-GPRC5D antibody or antigen binding fragment thereof provided herein has an IgG1 isotypes and includes one or more amino acid substitutions selected from the group consisting of N297A, N297Q, N297G, L235E, L234A, L235A, L234F, P331S and any combination thereof. In some embodiments, the anti-GPRC5D antibody or antigen binding fragment thereof provided herein has an IgG2 isotypes, and includes one or more amino acid substitutions selected from the group consisting of H268Q, V309L, A330S, P331S, V234A, G237A, P238S, H268A and any combination thereof (e.g. H268Q/V309L/A330S/P331S, V234A/G237A/P238S/H268A/V309L/A330S/P331S). In some embodiments, the anti-GPRC5D antibody or antigen binding fragment thereof provided herein has an IgG4 isotypes and includes one or more amino acid substitutions selected from the group consisting of N297A, N297Q, N297G, L235E, L234A, L235A, and any combination thereof. In some embodiments, the anti-GPRC5D antibody or antigen binding fragment thereof provided herein has an IgG2/IgG4 cross-isootype. Examples of the IgG2/IgG4 cross-isotype are described in Rother RP et al., Nat Biotechnol, 25:1256-1264 (2007).

In some embodiments, the anti-GPRC5D antibody or antigen binding fragment thereof provided herein has an IgG1 isotype and includes one or more amino acid substitutions at one or more of sites 234, 235 and 331. In some embodiments, the anti-GPRC5D antibody or antigen binding fragment thereof provided herein has an IgG1 isotype and include triple mutations L234F/L235E/P331S in the Fc region.

In some embodiments, the anti-GPRC5D antibody or antigen binding fragment thereof provided herein has increased ADCC and/or increased affinity to Fcγ receptor, and includes one or more amino acid substitutions at one or more of positions 238, 239, 248, 249, 252, 254, 255, 256, 258, 265, 267, 268, 269, 270, 272, 276, 278, 280, 283, 285, 286, 289, 290, 292, 293, 294, 295, 296, 298, 301, 303, 305, 307, 309, 312, 315, 320, 322, 324, 326, 327, 329, 330, 331, 333, 334, 335, 337, 338, 340, 360, 373, 376, 378, 382, 388, 389, 398, 414, 416, 419, 430, 434, 435, 437, 438 or 439 (see WO 00/42072 to Presta). Specific mutations at positions 256, 290, 298, 333, 334 and 339 have been shown to improve the binding to FcγRIII. In addition, the following combination of mutations are shown to improve the binding to FcγRIII: T256A/S298A, S298A/E333A, S298A/K224A, and S298A/E333A/K334A.

In some embodiments, the anti-GPRC5D antibody or antigen binding fragment thereof includes one or more amino acid substitutions that improve the pH-dependent binding to neonatal Fc receptor (FcRn). Such variants have an extended pharmacokinetic half-life because they bind to FcRn at an acidic pH to prevent them from being degraded in lysosomes, and then translocated and released from cells. Methods of engineering the antibody and antigen binding fragment thereof to improve its binding affinity to FcRn are well known in the art, see, for example Vaughn, D. et al., Structure, 6(1): 63-73, 1998; Kontermann, R. et al., Antibody Engineering, Vol. 1, Chapter 27: Engineering of the Fc region for improved PK, Springer, 2010; Yeung, Y. et al., Cancer Research, 70: 3269-3277 (2010); and Hinton, P. et al., J Immunol., 176:346-356 (2006).

The antibody or antigen binding fragment thereof provided herein may be a monoclonal antibody, a polyclonal antibody, a humanized antibody, a chimeric antibody, a recombinant antibody, a bispecific antibody, a multi-specific antibody, a divalent antibody or an anti-idiotypic antibody. The recombinant antibody is an antibody produced *in vitro* by a recombinant method rather than in animals.

### Conjugates

In some embodiments, the anti-GPRC5D antibody or antigen binding fragment thereof further comprises a conjugate moiety. The conjugate moiety can be linked to the antibody or antigen binding fragment thereof. The conjugate moiety is a moiety that can be linked to the antibody or antigen binding fragment thereof. It is considered that various conjugation moieties can be linked to the antibody or antigen binding fragment thereof provided herein (see, for example, "Conjugate Vaccines", Contributions to microbiology and immunology, J.M. Cruse and R.E.Lewis, Jr (ed.), Cage Press (New York) (1989)). These conjugation moieties can be linked to the antibody or antigen binding fragment thereof by covalent binding, affinity binding, intercalation, coordination, recombination, association, blending or addition, and other methods.

In some embodiments, the antibody and antigen binding fragment thereof disclosed herein can be engineered to contain specific sites other than the epitope conjugate moiety that can be used to bind to one or more conjugation moieties. For example, such sites may contain one or more reactive amino acid residues, such as, for example, cysteine or histidine residues, to facilitate the covalent attachment to the conjugate moiety.

In some embodiments, the antibody can be linked to the conjugate moiety indirectly or through another conjugate moiety. For example, the antibody or antigen binding fragment thereof can bind to biotin and then indirectly bind to a second conjugate that binds to avidin. The conjugate may be a clearance modulator, a toxin (such as a chemotherapeutic agent), a detectable label (such as a radioisotope, lanthanide, a luminescent label, a fluorescent label or an enzyme-substrate label) or a purification moiety.

The "toxin" can be any agent that is harmful to cells or can destroy or kill cells. Examples of toxins include, but are not limited to, taxol, cytochalasin B, gramicidin D, ethidium bromide, emetine, mitomycin, etoposide, tenoposide, vincristine, MMAE, MMAF, DM1, vinblastine, colchicin, doxorubicin, daunorubicin, dihydroxy anthracin dione, mitoxantrone, mithramycin, actinomycin, 1-dehydrotestosterone, glucocorticoid, procaine, tetracaine, lidocaine, propranolol, puromycin and anologs thereof, antimetabolites (such as methotrexate, 6- mercaptopurine, 6-thioguanine, cytarabine, and 5-fluorouracil decarbazine), alkylating agents (for example, mechlorethamine, thioepa chlorambucil, melphalan, carmustine (BSNU) and lomustine (CCNU), cyclophosphamide, busulfan, dibromomannitol, streptozotocin, mitomycin C, cis-diamminedichloridoplatinum (II) (DDP), and cisplatin), anthracycline (such as daunomycin) and doxorubicin), antibiotics (such as dactinomycin (formly known as actinomycin)), bleomycin, mithramycin and anthramycin (AMC) ), antimitotic agents (such as vincristine and vinblastine), topoisomerase inhibitors, and tubulin-binding agents.

Examples of detectable labels may include fluorescent labels (e.g., fluorescein, rhodamine, dansyl, phycoerythrin or Texas Red), enzyme-substrate labels (e.g., horseradish peroxidase, alkaline phosphatase, luciferase, glucoamylase, lysozyme, glucose oxidase or β-D-galactosidase), radioactive isotopes (e.g., ¹²³I, ¹²⁴I, ¹²⁵I, ¹³¹I, ³⁵S, ³H, ¹¹¹In, ¹¹²In, ¹⁴C, ⁶⁴Cu, ⁶⁷Cu, ⁸⁶Y, ⁸⁸Y, ⁹⁰Y, ¹⁷⁷Lu, ²¹¹At, ¹⁸⁶Re, ¹⁸⁸Re, ¹⁵³Sm, ²¹²Bi, ³²P, and other lanthanides), luminescent labels, chromogenic moieties, digoxigenin, biotin/avidin, DNA molecules or gold labels for detection.

In some embodiments, the conjugate moiety can be a clearance modulator that helps to increase the half-life of the antibody. Illustrative examples include water-soluble polymers such as PEG, carboxymethyl cellulose, dextran, polyvinyl alcohol, polyvinylpyrrolidone, ethylene glycol/propylene glycol copolymers, and the like. The polymer may have any molecular weight and can be branched or non-branched. The number of polymer molecules linked to the antibody may vary, and if more than one polymer molecule is linked, they can be the same or different molecules.

In some embodiments, the conjugate moiety can be a purification moiety, such as magnetic beads.

In some embodiments, the antibody and antigen binding fragment provided herein is used as the base of the conjugate.

### Chimeric antigen receptor (CAR) composition

The present application also provides a chimeric antigen receptor (CAR), which comprises an anti-GPRC5D antigen binding domain provided in the present application and an immune cell activation domain. The chimeric antigen receptor (CAR) is an engineered chimeric receptor, which combines the antigen binding domain of an antibody with one or more signal transduction domains for immune cell activation. Suitable immune cells may include, but are not limited to, T cells and natural killer (NK) cells. Immune cells such as T cells and natural killer (NK) cells can be genetically engineered to express CAR. T cells expressing CAR are called CAR-T cells. NK cells expressing CAR are called CAR-NK cells. CAR can mediate antigen-specific cellular immune activity in T cells or NK cells, so that CAR-T or CAR-NK cells can eliminate cells (such as tumor cells) expressing the target antigen. In one embodiment, the CAR-T or CAR-NK cells provided herein bind to GPRC5D expressed on, for example, cancer cells, resulting in the proliferation and/or activation of the CAR-T or CAR-NK cells. The activated CAR-T cells or CAR-NK cells can release cytotoxic factors such as perforin, granzyme and granulysin, and initiate the cell lysis and/or apoptosis of cancer cells.

In some embodiments, the CAR comprises an anti-GPRC5D antigen-binding domain, a transmembrane domain, and an immune cell activation domain, where the antigen-binding domain specifically binds to GPRC5D and comprises the antigen-binding fragment of the antibody provided herein. In some embodiments, the CAR further comprises a co-stimulatory signal transduction region, which is optionally linked to the transmembrane domain and the immune cell activation domain, respectively.

In some embodiments, the anti-GPRC5D antigen-binding domain of CAR contains one or more CDR sequences provided herein, one or more heavy chain variable domains or light chain variable domains provided herein, or one or more antigen-binding fragments derived from any anti-GPRC5D antibody provided herein.

In some embodiments, derivation from the same species is beneficial to the antigen binding domain, and the CAR will eventually be used in the same species. For example, when used in humans, the use of a human antibody- or humanized antibody-derived antigen binding domain in the CAR is beneficial. In some embodiments, the antigen binding domain contains a single-chain variable fragment (scFv). In some embodiments, the antigen binding domains can exist in many other forms, including, for example, Fv, Fab and (Fab')2, and a bifunctional (*i.e.*, bispecific) hybrid antibody fragment (for example, Lanzavecchia et al., Eur. J. Immunol. 17, 105(1987)). In some embodiments, the antigen binding domain comprises Fab or scFv.

In some embodiments, the CAR comprises a transmembrane domain fused to the extracellular antigen binding domain of the CAR. When expressed in cells, the anti-GPRC5D antigen binding domain is located outside the cell, and the immune cell activation domain is inside the cell. In one embodiment, the transmembrane domain can be selected such that it is naturally associated with a domain in the CAR.

The transmembrane domain in the CAR provided herein can be derived from a transmembrane domain of any transmembrane protein, such as the transmembrane domains of α, β or ζ chain derived from a T cell receptor, CD28, CD3ε, CD45, CD4, CD5, CD8, CD9, CD16, CD22, CD33, CD37, CD64, CD80, CD86, CD134, CD137 and CD154. In some embodiments, the transmembrane domain of the CAR can also be a variety of human hinges, such as human Ig (immunoglobulin) hinges.

In addition, the transmembrane domain of the CAR provided herein can also be synthetic, for example, it mainly contains a hydrophobic residue (e.g., leucine and valine). In one embodiment, a triplet of phenylalanine, tryptophan and valine is included at each terminus of the synthesized transmembrane domain. Optionally, a short oligopeptide linker or polypeptide linker having 2 and 10 amino acids in length can form the connection between the transmembrane domain and the intracellular signal transduction domain of CAR. The glycine-serine dimer provides a particularly suitable linker.

The co-stimulatory signal transduction region in CAR provided herein can function in an antigen-independent manner to mediate T cell activation or NK cell activation, and can be a co-stimulatory molecule required by lymphocytes for effective response to antigens. Exemplary co-stimulatory molecules include those derived from CD27, CD28, 4-1BB(CD137), OX40, CD30, CD40, PD-1, ICOS, lymphocyte function-related antigen-1(LFA-1), CD2, CD7, LIGHT, NKG2C, B7-H3, and ligands that specifically bind to CD83.

The immune cell activation domain of CAR provided herein can be a T cell activation domain. The T cell activation domain can be linked to the transmembrane domain or to the co-stimulatory signal transduction domain. The T cell activation domain can contain a TCR signal transduction domain. The TCR signal transduction domain can activate T cells expressing CAR to effect at least one normal TCR effector function of T cells, such as cytolytic activity or auxiliary activity including cytokine secretion. The TCR signal transduction domain can be a full-length natural intracellular signal transduction domain, or a fragment thereof that is sufficient to conduct a TCR effector functional signal. Exemplary intracellular signal transduction domains that can be used for CAR provided herein include the cytoplasmic sequences of T cell receptors (TCRs) and co-receptors, as well as any derivatives or variants of these sequences and any synthetic sequences with the same functions and abilities. The co-receptors jointly initiate signal transduction after CAR activation.

The TCR signal transduction domain that function by stimulation may contain a signal transduction motif, which is called immune receptor tyrosine-based activation motif or ITAM. Examples of ITAM containing a TCR signal transduction domain and useful in the CAR provided herein include those ITAM derived from CD3ζ, TCRζ, FcRγ, FcRβ, CD3γ, CD3δ, CD3ε, CD5, CD22, CD79a, CD79b and CD66d. In some embodiments, the TCR signal transduction domain comprises a cytoplasmic signal transduction sequence derived from CD3-ζ.

In another aspect, the present application provides a nucleic acid encoding the chimeric antigen receptor (CAR) as described in the present application. In another aspect, the present application provides a cell comprising the nucleic acid sequence provided in the present application that encodes the chimeric antigen receptor (CAR) as described in the present application. In another aspect, the present application provides a genetically modified cell expressing the chimeric antigen receptor (CAR) as described in the present application.

In one embodiment, the cell is autologous T cells or autologous NK cells. As used herein, "autologous" refers to any material derived from the same individual which is then reintroduced into that individual. In one embodiment, the cells are allogeneic T cells or allogeneic NK cells.

### Polynucleotide and recombination method

The present application provides an isolated polynucleotide encoding the anti-GPRC5D antibody and antigen-binding fragment thereof. In another aspect, the present application provides a nucleic acid encoding the chimeric antigen receptor (CAR) as described in the present application. As used herein, the term "nucleic acid" or "polynucleotide" refers to a single-stranded or double-stranded deoxyribonucleic acid (DNA) or ribonucleic acid (RNA) and polymers thereof. In some embodiments, the isolated polynucleotide encodes the variable region of the exemplary antibody provided herein. Unless otherwise indicated, a specific polynucleotide sequence also implicitly covers a conservatively modified variant thereof (for example, with degenerate codon substitution), an allele, an ortholog, SNP and a complementary sequence, as well as an explicitly indicated sequence. Specifically, degenerate codon substitution can be achieved by producing a sequence in which the third position of one or more selected (or all) codons is replaced by a mixed base and/or a deoxyinosine residue (see Batzer et al., Nucleic Acids Res., 19:5081 (1991); Ohtsuka et al., J. Biol. Chem., 260:2605-2608 (1985); and Rossolini et al., Mol. Cell. Probes, 8:91-98 (1994)).

DNA encoding a monoclonal antibody can be easily isolated and sequenced by using a conventional procedure (for example, by using an oligonucleotide probe that can specifically bind to a genes encoding the heavy chain and light chain of the antibody). The coding DNA can also be obtained by synthesis.

The isolated polynucleotide encoding the anti-GPRC5D antibody and antigen binding fragment thereof (*e.g.*, comprising the sequence shown in Tables 1-4) or CAR can be inserted into a vector by a recombinant technique known in the art, for further cloning (DNA amplification) or expression. Many vectors can be used. The vector component generally includes, but is not limited to, one or more of a signal sequence, a replication origin, one or more marker genes, an enhancer elements, a promoter (such as SV40, CMV, and EF-1α) and a transcription termination sequence.

The present application provides an expression vector comprising the isolated polynucleotide provided herein. In some embodiments, the polynucleotide provided herein encodes the antibody or antigen binding fragment thereof, at least one promoter (e.g., SV40, CMV, and EF-1α) operably linked to a nucleic acid sequence, and at least one selection marker. Examples of vectors include, but are not limited to, retrovirus (including lentivirus), adenovirus, adeno-associated virus, herpes virus (such as herpes simplex virus), poxvirus, baculovirus, papillomavirus, papovavirus (such as SV40), λ phage and M13 phage, and plasmids pcDNA3.3, pMD18-T, pOptivec, pCMV, pEGFP, pIRES, pQD-Hyg-GSeu, pALTER, pBAD, pcDNA, pCal, pL, pET, pGEMEX, pGEX, pCI, pEGFT, pSV2, pFUSE, pVITRO, pVIVO, pMAL, pMONO, pSELECT, pUNO, pDUO, Psg5L, pBABE, pWPXL, pBI, p15TV-L, pPro18, pTD, pRS10, pLexA, pACT2.2, pCMV-SCRIPT.RTM., pCDM8, pCDNA1.1/amp, pcDNA3.1, pRc/RSV, PCR 2.1, pEF-1, pFB, pSG5, pXT1, pCDEF3, pSVSPORT, pEF-Bos, and others.

A vector including a polynucleotide sequence encoding the antibody or antigen binding fragment thereof can be introduced into a host cell for cloning or gene expression. Suitable host cells for cloning or expressing DNA in the vector herein are prokaryotes, yeasts or higher eukaryotes as described above. Suitable prokaryotes for this purpose include eubacteria, such as Gram-negative or Gram-positive organisms, such as *Enterobacteriaceae* such as *Escherichia, e.g., E. coli, Enterobacter, Erwinia, Klebsiella, Proteus, Salmonella, e.g., Salmonella typhimurium, Serratia, e.g., Serratia marcescans,* and *Shigella,* as well as *Bacilli* such as *B. subtilis* and *B. licheniformis, Pseudomonas* such as P. *aeruginosa,* and *Streptomyces.*

In addition to prokaryotes, eukaryotic microorganisms such as filamentous fungi or yeast are suitable cloning or expression hosts for antibody coding vectors. Saccharomyces cerevisiae or common baking yeast is the most commonly used lower eukaryotic host microorganisms. However, many other genera, species and strains are commonly used and useful herein, for example, *Schizosaccharomyces pombe; Kluyveromyces,* for example, *K. lactis, K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906), *K. thermotolerans* and *K. marxianus; yarrowia* (EP 402,226); *Pichia pastoris* (EP 183,070*); Candida; Trichoderma reesia* (EP 244,234); *Neurospora crassa; Schwanniomyces,* for example, *Schwanniomyces occidentalis;* and filamentous fungi, for example, *Neurospora, Penicillium, Tolypocladium* and *Aspergillus* hosts, such as *A. nidulans* and *A. niger.*

Suitable host cells expressing the glycosylated antibody or antigen binding fragment thereof provided herein are derived from multicellular organisms. Examples of invertebrate cells include plant and insect cells. A variety of baculovirus strains and variants, as well as corresponding allowable insect host cells from the following hosts are identified: *Spodoptera frugiperda* (caterpillar), *Aedes aegypti)* (mosquito), *Aedes albopictus* (mosquito), *Drosophila melanogaster* (fruit flies) and *Bombyx mori.* A variety of virus strains for transfection are publicly available, such as L-1 variant of *Autographa californica* NPV and Bm-5 virus strain of Bombyx mori NPV, and such viruses can be used as viruses herein according to the present invention, especially for transfection of Spodoptera frugiperda cells. Plant cell cultures of cotton, corn, potato, soybean, Petunia, tomato and tobacco can also be used as hosts.

However, vertebrate cells receive greatest interest, and the culture and proliferation of vertebrate cells (tissue culture) has become a routine procedure. Examples of useful mammalian host cell lines are the monkey kidney cell line CV1 (COS-7, ATCC CRL 1651) transformed by SV40; human embryonic kidney cell line (293 or 293 cells subcloned to grow in suspension culture); Graham et al., J. Gen Virol., 36:59 (1977)); baby hamster kidney cells (BHK, ATCC CCL 10); Chinese hamster ovary cells/-DHFR (CHO, Urlaub et al., Proc Natl Acad Sci USA, 77:4216 (1980)); mouse support cells (TM4, Mather, Biol. Reprod., 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human hepatocytes (Hep G2, HB 8065); mouse breast tumor cells (MMT 060562, ATCC CCL51); TRI cells (Mather et al., Annals N.Y. Acad. Sci., 383:44-68 (1982)); MRC 5 cells; FS4 cells; and human hepatic tumor cell line (Hep G2). In some preferred embodiments, the host cells are mammalian culture cell lines, such as CHO, BHK, NS0, 293 and their derivatives.

The host cells are transformed with the above expression or cloning vector for producing the anti-GPRC5D antibody or antigen binding fragment thereof, and cultured in a conventional nutrient medium modified as needed to induce the promoter, select a transformant or amplify a gene encoding the desired sequence. In another embodiment, the antibody can be produced by homologous recombination known in the art. In some embodiments, the host cell can produce the antibody or antigen binding fragment thereof provided herein.

The host cell used to produce the antibody or antigen binding fragment thereof provided herein can be cultured in various media. Commercial media, such as Ham's F10 (Sigma), minimum essential medium (MEM) (Sigma), RPMI-1640 (Sigma) and Dulbecco's Modified Eagle's Medium (DMEM, Sigma) are suitable for culturing the host cells. In addition, any medium described in Ham et al., Meth. Enz., 58:44(1979); Barnes et al., Anal. Biochem.,102:255 (1980); US Patent Nos. 4,767,704, 4,657,866, 4,927,762, 4,560,655 or 5,122,469; WO90/03430; WO 87/00195; or US Reissue Patent No. 30,985 can be used as the culture medium for the host cells. Any of these media can be supplemented with a hormone and/or other growth factors (such as insulin, transferrin or epidermal growth factor), a salt (such as sodium chloride, a calcium salt, a magnesium salt and a phosphate), a buffer (such as HEPES), a nucleotide (such as adenosine and thymidine), an antibiotic (such as GENTAMYCIN^{™}), a trace element (defined as inorganic compounds that usually exist at a final concentration in the micromolar range), and glucose or an equivalent energy source as required. Any other necessary supplements may also be included in appropriate concentrations known to those skilled in the art. The culture conditions, such as temperature, and pH, etc., are those previously used for the expression of host cells, and will be obvious to those of ordinary skill in the art.

When the recombinant technology is used, the antibody can be produced in the periplasmic space in the cells, or secreted directly into the culture medium. If the antibody is produced in the cells, then the host cells or the particle debris of lyzed fragments are removed for example by centrifugation or ultrafiltration in a first step. Carter et al., Bio/Technology, 10: 163-167 (1992) describes a procedure for isolating an antibody secreted into the periplasmic space of Escherichia coli. Briefly, in the presence of sodium acetate (pH 3.5), EDTA and benzyl sulfonyl fluoride (PMSF), the cell paste is thawed in about 30 minutes. The cell debris can be removed by centrifugation. When an antibody is secreted into the culture medium, the supernatant from such expression systems is generally concentrated by using a commercially available protein concentration filter, such as Pellicon ultrafiltration unit from Amicon or Millipore. A protease inhibitor, such as PMSF, may be included in any of the aforementioned steps to inhibit the proteolysis, and antibiotics may be included to prevent the growth of foreign pollutants.

The anti-GPRC5D antibody and antigen binding fragment thereof prepared from cells can be purified by, for example, hydroxyapatite chromatography, gel electrophoresis, dialysis, DEAE-cellulose ion exchange chromatography, precipitation with ammonium sulfate, salt precipitation and affinity chromatography, where the affinity chromatography is the preferred purification technique.

In some embodiments, protein A immobilized on the solid phase is used for immunoaffinity purification of the antibody and antigen binding fragment thereof. The suitability of protein A as an affinity ligand depends on the species and isotypes of any immunoglobulin Fc domain present in the antibody. Protein A can be used to purify antibodies based on human γ1, γ2 or γ4 heavy chain (Lindmark et al., J. Immunol. Meth., 62:1-13 (1983)). Protein G is recommended for all mouse isotypes and human γ3 (Guss et al., EMBO J., 5:1567 1575 (1986)). The matrix to which the affinity ligand is attached is most commonly agarose, but other matrices can also be used. Mechanically stable matrices, such as controlled-pore glass or poly(styrene-divinyl benzene) allow for faster flow rate and shorter treatment time than agarose. Bakerbond ABX^{™} resin (Phillipsburg, N.J., J. T. Baker) can be used for purification when the antibody includes a CH3 domain. Other techniques for protein purification can also be used, such as fractionation on ion exchange column, ethanol precipitation, reversed-phase HPLC, chromatography on silica gel, chromatography on heparin SEPHAROSE^{™}, chromatography on anion or cation exchange resin (such as poly-aspartate column), chromatofocusing, SDS-PAGE, and precipitation with ammonium sulfate, depending on the antibody to be recovered.

After any preliminary purification step, the mixture containing the antibody of interest and contaminants can be subjected to low-pH hydrophobic interaction chromatography using an elution buffer with a pH between about 2.5 and 4.5, preferably at a low salt concentration (*e.g.*, about 0-0.25M salt).

### Pharmaceutical composition

The present application further provides a pharmaceutical composition, which comprises the anti-GPRC5D antibody or antigen-binding fragment thereof as described in the present application, the polynucleotide as described in the present application, the vector as described in the present application, the conjugate as described in the present application, the nucleic acid having a nucleic acid sequence encoding the chimeric antigen receptor (CAR) of the present application as described in the present application, the cell expressing the chimeric antigen receptor (CAR) of the present application as described in the present application, and one or more pharmaceutically acceptable carriers.

The pharmaceutically acceptable carrier for use in the pharmaceutical composition disclosed herein may include, for example, a pharmaceutically acceptable liquid, a gel or a solid carrier, an aqueous vehicle, a non-aqueous vehicle, an antimicrobial agent, an isotonic agent, a buffer, an antioxidant, an anesthetic, a suspending/dispersing agent, a sequestering/chelating agent, a diluent, an adjuvant, an excipient, a non-toxic aid, and other components known in the art, or various combinations thereof.

Suitable components may include, for example, antioxidants, fillers, binders, disintegrants, buffers, preservatives, lubricants, flavoring agents, thickeners, colorants, emulsifiers or stabilizers such as sugars and cyclodextrins. Suitable antioxidant may include, for example, methionine, ascorbic acid, EDTA, sodium thiosulfate, platinum, catalase, citric acid, cysteine, thioglycerol, thioglycolic acid, thiosorbitol, butylated hydroxanisol, butylated hydroxytoluene and/or propyl gallate. As disclosed herein, the composition comprising the antibody or antigen binding fragment and the conjugate as provided herein comprises one or more antioxidants, such as methionine, to reduce the oxidation of the antibody or antigen binding fragment. This reduction in oxidation prevents or reduces the loss of binding affinity, thereby improving the antibody stability and maximizing the storage time. Therefore, in some embodiments, a composition comprising one or more antibodies or antigen binding fragments as disclosed herein and one or more antioxidants such as methionine is provided. A method for preventing the oxidation, extending the storage time, and/or improving the efficacy of the antibody or antigen binding fragment as provided herein by mixing the antibody or antigen binding fragment with one or more antioxidants such as methionine is also provided.

For further explanation, the pharmaceutically acceptable carrier may include, for example, an aqueous vehicle such as sodium chloride injection, Ringer's injection, isotonic dextrose injection, sterile water injection, or dextrose and lactate in Ringer's injection; a non-aqueous vehicle, such as a plant-derived non-volatile oil, cottonseed oil, corn oil, sesame oil or peanut oil; an antimicrobial agent that inhibits the concentration of bacteria or fungi; an isotonic agent, such as sodium chloride or dextrose; a buffer, such as a phosphate or citrate buffer; an antioxidant, such as sodium bisulfate; a local anesthetic, such as procaine hydrochloride; a suspending and dispersing agent, such as sodium carboxymethyl cellulose, hydroxypropyl methylcellulose or polyvinylpyrrolidone; an emulsifier, such as polysorbate 80 (TWEEN-80); a chelating agent, such as ethylenediamine tetraacetic acid (EDTA) or ethylene glycol tetraacetic acid (EGTA), ethanol, polyethylene glycol, propylene glycol, sodium hydroxide, hydrochloric acid, citric acid or lactic acid. The antimicrobial agent used as a carrier can be added to the pharmaceutical composition in a multi-dose container. The antimicrobial agent includes phenol or cresol, a mercuric agent, benzyl alcohol, chlorobutanol, methyl and propyl p-hydroxybenzoate, thiomersal, benzalkonium chloride and benzethonium chloride. Suitable excipients may include, for example, water, physiological saline, dextrose, glycerol or ethanol. Suitable nontoxic aids may include, for example, a wetting agent or an emulsifier, a pH buffer, a stabilizer, a solubility enhancer or agents such as sodium acetate, sorbitan monolaurate, triethanolamine oleate or cyclodextrin.

The pharmaceutical composition can be in the form of a liquid solution, a suspension, an emulsion, pills, capsules, tablets, a sustained release preparation or a powder. An oral preparation can contain a standard carrier, such as pharmaceutical-grade mannitol, lactose, starch, magnesium stearate, polyvinylpyrrolidone, saccharin sodium, cellulose, and magnesium carbonate, etc.

In some embodiments, the pharmaceutical composition is prepared into an injectable composition. The injectable pharmaceutical composition can be prepared into any conventional form, for example, a liquid solution, a suspension, an emulsion or a solid form suitable for producing a liquid solution, a suspension or an emulsion. Preparations for injection may contain sterile and/or pyrogen-free solutions that can be used for injection immediately; sterile dry soluble products that are prepared to combine with a solvent only before use, such as freeze-dried powder, including subcutaneous tablets; sterile suspension ready for injection; sterile dry insoluble products that are prepared to combine with a vehicle only before use; and sterile and/or pyrogen-free emulsions. The solution can be aqueous or non-aqueous.

In some embodiments, a unit dose of a parenteral preparation is packed in an ampoule, a vial or a syringe with a needle. All preparations for parenteral administration should be sterile and pyrogen-free, as known and practiced in the art.

In some embodiments, the sterile freeze-dried powder is prepared by dissolving the antibody or antigen binding fragment as disclosed herein in a suitable solvent. The solvent may contain an excipient that improves the stability of the powder or a reconstitution solution prepared from the powder or other pharmacological components. Excipients that can be used include, but are not limited to, water, dextrose, sorbitol, fructose, corn syrup, xylitol, glycerol, glucose, sucrose or other suitable reagents. The solvent may contain a buffer, such as a nitrate, sodium phosphate or potassium phosphate, or other such buffers known to those skilled in the art. In one embodiment, the buffer is at about a neutral pH. Then the solution is sterilized and filtered under the standard conditions known to those skilled in the art, and then freeze-dried to obtain the required preparation. In one embodiment, the resulting solution is dispensed into vials for lyophilization. Each vial can contain a single dose or multiple doses of the anti-GPRC5D antibody or antigen binding fragment thereof or its composition. It is acceptable that the vial is overfilled with a small amount (for example, about 10%) excess of the required single dose or a group of doses, so as to facilitate accurate sampling and accurate administration. The freeze-dried powder can be stored under appropriate conditions, such as at about 4°C to room temperature.

The freeze-dried powder reconstituted with water for injection provides a formulation for parenteral administration. In one embodiment, sterile and/or pyrogen-free water or other suitable liquid carriers are added to the lyophilized powder for reconstitution. The exact amount depends on the chosen therapy given and can be determined empirically.

### Method of use

The present application also provides a method for treating a GPRC5D-related disease or condition in a subject, which comprises administering, to the subject, a therapeutically effective amount of an antibody or an antigen binding fragment thereof provided herein or a pharmaceutical composition provided herein.

In another aspect, the present application provides a method for stimulating an immune cell (such as T cell or NK cell)-mediated immune response to cells or tissues expressing GPRC5D in a mammal, the method comprising administering an effective amount of genetically modified cells to the mammal to express the CAR in the present application.

In another aspect, the present application provides a method for treating a mammal having a GPRC5D-related disease or condition, the method comprising administering an effective amount of the cell provided in the present application to the mammal, to treat the mammal.

In some embodiments, the GPRC5D-related disease or condition is characterized by the expression or overexpression of GPRC5D. The overexpression of GPRC5D has been confirmed in several autoimmune diseases, including myeloma.

In some embodiments, the GPRC5D-related disease or condition includes, but is not limited to, cancers and other hyperproliferative diseases, immune diseases and inflammation.

In some embodiments, the cancers and other hyperproliferative diseases include benign or malignant tumors, leukemia and malignant lymphoid tumors. Examples include neuron, glial cell, astrocyte, hypothalamus, glandular cell, macrophage, epithelial cell, endothelial cell, and interstitial malignant tumors, according to cell types with cancers or hyperproliferative diseases. Depending on the organ/site suffering from the cancer or hyperproliferative disease, examples include: head, neck, eyes, mouth, throat, esophagus, chest, skin, bone, lung, colon, rectum, colon, stomach, spleen, kidney, skeletal muscle, subcutaneous tissue, metastatic melanoma, endometrium, prostate, breast, ovary, testis, thyroid, blood, lymph node, kidney, liver, pancreas, brain or central nervous system etc.

In some embodiments, the immune diseases and/or inflammations include alopecia areata, ankylosing spondylitis, antiphospholipid syndrome, autoimmune Addison's disease, adrenal autoimmune diseases, autoimmune hemolytic anemia, autoimmune hepatitis, autoimmune oophoritis and orchitis, Sjogren's syndrome, psoriasis, atherosclerosis, diabetes and other retinopathy, retrolental fibroplasia, age-related macular degeneration, neovascular glaucoma, hemangioma, thyroid hyperplasia (including Graves' disease), corneal and other tissue transplantation, chronic inflammation, sepsis, rheumatoid arthritis, peritonitis, Crohn's disease, reperfusion injury, sepsis, endotoxic shock, cystic fibrosis, endocarditis, psoriasis, arthritis (such as psoriatic arthritis), anaphylactic shock, organ ischemia, reperfusion injury, spinal cord injury and allograft rejection, autoimmune thrombocytopenia, Behcet's disease, bullous pemphigoid, cardiomyopathy, dermatitis herpetiformis, chronic fatigue immune dysfunction syndrome (CFIDS), chronic inflammatory demyelinating polyneuropathy, Churg-Strauss syndrome, cicatricial pemphigoid, CREST syndrome, cold agglutinin disease, discoid lupus erythematosus, primary mixed cryoglobulinemia, fibromyalgia, fibromyositis, glomerulonephritis, Guillain-Barre syndrome, Hashimoto's thyroiditis, idiopathic pulmonary fibrosis, idiopathic thrombocytopenic purpura (ITP), IgA neuropathy, juvenile arthritis, lichen planus, lupus erythematosus, Meniere's disease, mixed connective tissue disease, multiple sclerosis, type 1 or immune-mediated diabetes, myasthenia gravis, pemphigus vulgaris, pernicious anemia, polyarthritis nodosa, hypertrichosis, multiple syndrome, polymyalgia rheumatica, polymyositis and dermatomyositis, primary agammaglobulinemia, primary biliary cirrhosis, psoriasis, psoriatic arthritis, Raynaud's phenomenon, Reiter's syndrome, rheumatoid arthritis, nodular disease, scleroderma, Sjogren's syndrome, stiff-person syndrome, systemic lupus erythematosus, lupus erythematosus, Takayasu's arteritis, temporal arteritis/giant cell arteritis, ulcerative colitis, uveitis, vasculitis, such as dermatitis herpetiformis-associated vasculitis, leukodermia and Wegener's granulomatosis. The inflammatory diseases may further include, but are not limited to, asthma, encephalitis, inflammatory bowel disease, chronic obstructive pulmonary disease (COPD), allergic diseases, septic shock, pulmonary fibrosis, undifferentiated spondyloarthritis, undifferentiated arthropathy, arthritis, inflammatory osteolysis, and chronic inflammation caused by chronic viral or bacterial infection.

In one embodiment, the GPRC5D-related disease or condition is cancers, especially multiple myeloma. In one embodiment, the GPRC5D-related disease or condition is an autoimmune disease, such as systemic lupus erythematosus and/or rheumatoid arthritis.

In some embodiments, the GPRC5D-related disease or condition is an GPRC5D expressing cancer. As used herein, the "GPRC5D expressing cancer" refers to any cancer or tumor that expresses GPRC5D on the surface of cancer cells. In some embodiments, the expression level of GPRC5D on cancer cells expressing GPRC5D is significantly higher than the expression level of GPRC5D on normal cells.

In some embodiments, the subject is identified as having cancer cells expressing GPRC5D. The presence and/or expression level of GPRC5D on cancer cells can be determined by various methods known in the art. A biological sample containing or suspected of containing cancer cells can be obtained from a subject. In some embodiments, the biological sample may be derived from cancer cells or cancer tissues. In some embodiments, the biological sample may be further treated to, for example, separate an analyte, such as a nucleic acid or protein. The presence and/or expression level of GPRC5D can be determined by, for example, quantitative fluorescence cell counting, immunohistochemistry (IHC), or nucleic acid-based methods. For example, a biological sample from a subject may be exposed to the anti-GPRC5D antibody or antigen binding fragment that binds to and detects the expressed GPRC5D protein. Alternatively, methods such as qPCR, reverse transcriptase PCR, microarray, SAGE, and FISH, etc. can also be used to detect GPRC5D at the nucleic acid expression level.

In one embodiment, the GPRC5D-related disease or condition includes, but is not limited to, breast cancer, multiple myeloma, Waldenstrom's macroglobulinemia, endometrial cancer, ovarian cancer, lung cancer, gastric cancer, prostate cancer, renal cancer, liver cancer, pancreatic cancer, colorectal cancer, esophageal cancer, bladder cancer, cervical cancer, hematological cancer, lymphoma or malignant melanoma that express GPRC5D.

The therapeutically effective amount of the antibody or antigen binding fragment or pharmaceutical composition as provided herein depends on various factors known in the art, such as the weight, age, past medical history, current medication, health status and possibility of cross-reaction, allergy, sensitivity and adverse side effects of the subjects, as well as the route of administration and the degree of disease development. As indicated by these and other situations or requirements, those skilled in the art (such as doctors or veterinarians) can reduce or increase the dosage proportionally.

In some embodiments, the antibody or antigen binding fragment or polypeptide complex as provided herein can be administered at a therapeutically effective dose of about 0.01 mg/kg to about 100 mg/kg. In some embodiments, the dosage administered can be changed during the treatment. For example, in some embodiments, the initial dosage may be higher than the subsequent dosage administered. In some embodiments, depending on the response of the subject, the administered dosage may be changed during the treatment.

The dosage regimen can be adjusted to provide the optimal desired response (e.g., therapeutic response). For example, a single dose may be administered, or several divided doses may be administered over time.

The antibody and antigen binding fragment disclosed herein can be administered through any route known in the art, such as, for example, parenteral (such as subcutaneous, intraperitoneal, intravenous (including intravenous infusion), intramuscular or intradermal injection) or non-parenteral (such as oral, nasal, intraocular, sublingual, rectal or topical) route.

In some embodiments, the antibody or antigen-binding fragment thereof disclosed herein can be administered alone or in combination with one or more additional therapeutic means or agents. For example, the antibody or antigen-binding fragment disclosed herein can be administered in combination with a second therapeutic agent *(e.g.,* chemotherapeutic agent, anti-cancer agent, radiotherapy, immunotherapy, anti-angiogenic agent, targeting therapy, cell therapy, gene therapy, hormone therapy, and palliative therapy), surgery for cancer treatment (e.g., tumor resection), or one or more treatments for complications caused by chemotherapy.

As used herein, the term "immunotherapy" refers to a type of therapy that stimulates the immune system to combat the diseases such as cancers or strengthens the immune system in a general way. The immunotherapy includes passive immunotherapy. The passive immunotherapy is carried out by delivering drugs (e.g., effector cells) with definite tumor immunoreactivity, which can directly or indirectly mediate the anti-tumor effect without relying on the complete immune system in the host (e.g., antibody therapy or CAR-T cell therapy). The immunotherapy may further include active immunotherapy. The treatment relies on the in-vivo stimulation of the endogenous immune system in the host by administering an immune response modulator to fight against diseased cells.

In some of these embodiments, the antibody or antigen binding fragment as disclosed herein administered in combination with one or more additional therapeutic agents can be administered simultaneously with the one or more additional therapeutic agents, and in some of these embodiments, the antibody or antigen binding fragment and the additional therapeutic agent(s) can be administered as part of the same pharmaceutical composition. However, the antibody or antigen binding fragment administered "in combination" with another therapeutic agent need not be administered with the agent simultaneously or in the same composition. The antibody or antigen binding fragment administered before or after another agent is considered to be administered "in combination" with the agent, as the phrase is used herein, even if the polypeptide complex and the second agent are administered by different routes. When possible, according to the schedule listed in the product information form of an additional therapeutic agent or according to the Physicians'Desk Reference 2003 (Physicians'Desk Reference, Edition 57; Medical Economics Company; ISBN: 1563634457; Edition 57 (Nov. 2002)) or a protocol well known in the art, the additional therapeutic agent is administered in combination with the antibody or antigen binding fragment disclosed herein.

The present application further provides a method for inhibiting the growth of cells expressing GPRC5D in vivo or in vitro, which comprises: contacting the cells expressing GPRC5D with the antibody or antigen-binding fragment thereof provided herein. In some embodiments, the present application provides a method for modulating the activity of GPRC5D in cells expressing GPRC5D, which comprises exposing the cells expressing GPRC5D to the antibody or antigen-binding fragment thereof provided herein.

In some embodiments, the present application provides a method for detecting the presence or level of GPRC5D in a sample, which comprises contacting the sample with the antibody or antigen binding fragment provided herein. In some embodiments, the method further comprises determining the presence or content of GPRC5D in the sample. In certain embodiments, the biological sample comprises cancer cells.

In some embodiments, the present application provides a method for diagnosing a GPRC5D-related disease or condition in a subject, which comprises: a) contacting a sample obtained from the subject with the antibody or antigen-binding fragment thereof provided herein; b) determining the presence or content of GPRC5D in the sample; and c) associating the presence or content of GPRC5D with the presence or state of the GPRC5D-related disease or condition in the subject.

In some embodiments, the present application provides a detection or therapeutic kit, which includes the antibody or antigen binding fragment thereof provided herein and optionally instructions for use with a detectable moiety. In some embodiments, the present application provides a kit comprising the antibody or antigen-binding fragment thereof provided herein optionally conjugated with a detectable moiety. The kit is suitable for detecting GPRC5D or for the treatment of GPRC5D-related diseases or conditions.

In some embodiments, the present application provides a kit, which includes the antibody or antigen binding fragment thereof provided herein and a second therapeutic agent. The kit can be used for treating, preventing and/or improving GPRC5D-related diseases.

In some embodiments, the present application also provides use of the antibody or antigen binding fragment thereof provided herein in manufacturing a medicament for treating a GPRC5D-related disease or condition in a subject and for manufacturing a diagnostic reagent for diagnosing a GPRC5D-related disease or condition.

The following examples are provided to better illustrate the claimed invention and should not be construed as limiting the scope of the invention. The particular compositions, materials and methods described below totally or partly fall within the scope of the present invention. These specific compositions, materials and methods are not intended to limit the present invention, but used to illustrate specific embodiments that fall within the scope of the present invention. Those skilled in the art can develop equivalent compositions, materials and methods with no need of fulfilling the inventiveness, without departing from the scope of the invention. It should be understood that many changes can be made to the procedures described herein within the scope of the present invention. The present inventors anticipates that such variations are included in the scope of the present invention.

### Examples:

### Example 1: Preparation of GPRC5D monoclonal antibody

In this example, a tumor cell line expressing GPRC5D was used to immunize mice to prepare a monoclonal antibody.

### 1.1 Construction of 293T-GPRC5D and CHOS-GPRCSD cell lines

The human GPRC5D (hGPRC5D) was over-expressed in HEK293 cells (ATCC) and CHOS cells (Invitrogen) by lentivirus infection (MOI=3-10, 5 µg/ml polybrene). 72 hrs after cell infection, the cells were added with a corresponding antibiotic and were continuously cultured for 2-4 weeks. The cells were expanded and frozen to obtain 2 over-expressed cell lines, HEK293-hGPRC5D and CHOS-hGPRC5D, which were used for subsequent immunological experiments.

### 1.2 Construction of control antibody GC5B596

The HC and LC plasmids of GC5B596 antibody were constructed with the following sequences, and then transferred into CHO cells and cultured. After harvest, the supernatant was purified by affinity chromatography to obtain the control antibody GC5B596.

**Table 5**

| Antibody | Sequence (the CDR region is underlined) |
|---|---|
| GC5B596 | Heavy chain_HC |
| | QVQLVQSGAEVKKPGASVKVSCKASGYSFTGYTMNWVRQAPGQG LEWMGLINPYNSDTNYAQKLQGRVTMTTDTSTSTAYMELRSLRSDD TAVYYCARVALRVALDYWGQGTLVTVSSASTKGPSVFPLAPCSRSTS ESTAALGCLVKDYFPEPVTVSWNSGALTSGVHTFPAVLQSSGLYSLSS VVTVPSSSLGTKTYTCNVDHKPSNTKVDKRVESKYGPPCPPCPAPEF LGGPSVFLFPPKPKDTLMISRTPEVTCVVVDVSQEDPEVQFNWYVD GVEVHNAKTKPREEQFNSTYRVVSVLTVLHQDWLNGKEYKCKVSN KGLPSSIEKTISKAKGQPREPQVYTLPPSQEEMTKNQVSLTCLVKGFY PSDIAVEWESNGQPENNYKTTPPVLDSDGSFFLYSRLTVDKSRWQEG NVFSCSVMHEALHNHYTQKSLSLSLGK (SEQ ID NO: 22) |
| | Light chain_LC |
| | DIQMTQSPSSLSASVGDRVTITCKASQNVATHVGWYQQKPGKAPKR LIYSASYRYSGVPSRFSGSGSGTEFTLTISNLQPEDFATYYCQQYNRY PYTFGQGTKLEIKRTVAAPSVFIFPPSDEQLKSGTASVVCLLNNFYPR EAKVQWKVDNALQSGNSQESVTEQDSKDSTYSLSSTLTLSKADYEK HKVYACEVTHQGLSSPVTKSFNRGEC (SEQ ID NO: 23) |

### 1.3 Mouse immunization/hybridoma fusion

To obtain an anti-human GPRC5D antibody, Balb/c mice (Beijing Weitong Lihua Laboratory Animal Technology Co., Ltd., strain code 216) were immunized with the constructed HEK293-GPRC5D cells overexpressing human GPRC5D. Complete Freund's adjuvant CFA (InvivoGen, article number vac-cfa-60) was used as an adjuvant in the primary immunization, and IFA (InvivoGen, article number vac-ifa-60) was used as an adjuvant in the subsequent immunization. The immunization route was subcutaneous multipoint. After multiple immunization, the spleen cells of immunized mice were fused with mouse myeloma cells SP2/0 by the polyethylene glycol method, and cultured in an HAT selective medium to obtain hybridoma cells that could express the antibody and proliferate indefinitely in vitro. The hybridoma cells were plated and cultured in a 96-well cell culture plate.

### 1.4 Cloning and screening of hybridoma

The antibody secreted by the hybridoma cells in the 96-well cell culture plate was tested for its binding ability to GPRC5D at a cell level. The cells highly expressing GPRC5D were cultured in a DMEM medium containing 10% FBS. The cells were digested with TrypLE, centrifuged and resuspended in a DPBS solution (FACS buffer, 4°C) containing 2% BSA. Then the cells were added to a 96-well plate with a U-shaped bottom in an amount of 5 x 10⁵ cells/50µl and placed in a low-adsorption 96-well plate with a round bottom. 50 µl of mouse hybridoma supernatant was added and incubated at 4°C for 1 hr. After centrifugation, the supernatant was removed. The cells were washed twice with a FACS buffer, and the secondary antibody (DyLight488 goat anti-human IgG, Abcam, ab97003) was added to each well and incubated at 4°C for 0.5 hr. After centrifugation, the supernatant was removed. The cells were washed twice with a FACS buffer, and the FACS buffer was added to each well to resuspend the cells. The fluorescence of the cells in the test plate was measured by flow cytometer (BD, model CantoII) to determine the binding of the hybridoma supernatant and the cells. Meanwhile, HEK293, the background cell used for constructing the GPRC5D overexpression cells, was used for the same binding analysis. Using the criteria of positive binding to HEK293-hGPRC5D and negative binding to HEK293 cell, the GPRC5D-binding positive clones were picked up and subcloned for 2-3 times.

### 1.5 Hybridoma sequencing/construction of recombinant expression vector

A ch-72C7 clone was obtained by screening. The picked hybridoma clone was sequenced according to the standard hybridoma sequencing method to obtain the heavy chain and light chain variable regions (VH and VL) of the picked clones. VH and VL were synthesized by whole gene synthesis, and human IgG1 and kappa constant region were linked. The heavy chain and light chain sequences were ligated into the pcDNA3.4 vector, expressed transiently in the 293 system and purified by Protein A/G. The obtained chimeric recombinant antibody was ultrafiltered and the buffer was replaced with a PBS solution. The sequencing results of ch-72C7 clone are shown in Table 6.

**Table 6**

| Clone | Sequence (the CDR region is underlined) |
|---|---|
| ch-72C7 | Heavy chain variable region_VH |
| | QIVLSQFPAIMSASPGEKVTMTCSASSSVSFMHWYQQKSGTSPKRW IYDTTKLASGVPARFTGSGSGTSYSLTISSMEAEDAATYYCQQWNS HPLTFGPGTKLELK (SEQ ID NO: 13) |
| | Light chain variable region_VL |
| | QVQLQQPGAELVKPGTSVKLSCKASGYPFTNYWMHWVRQRPGQ GLEWIGEINPSNGRTNYNEKFKSLATLTLDKSSSTAYMHLNSLTSED SAVYYCARGFAYWGQGTLVTVSA (SEQ ID NO: 12) |

### Example 2: FACS test for antigen binding

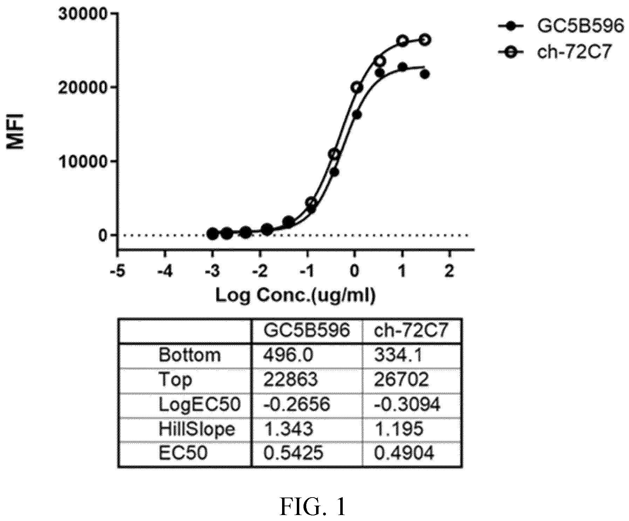

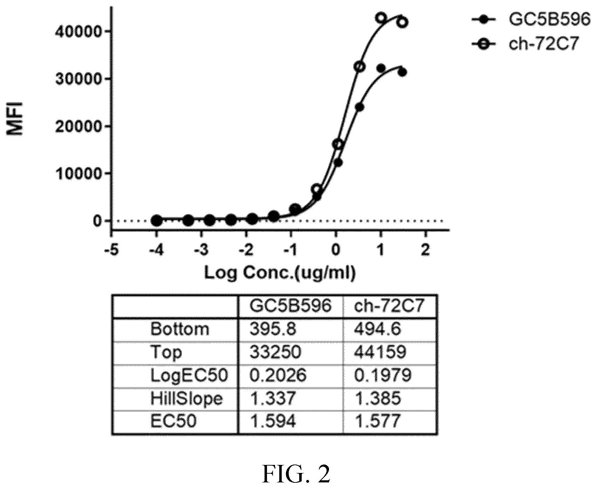

After centrifugation, HEK293-hCD22GPRC5D and CHOS-hGPRC5D cells expressing hGPRC5D were resuspended in a DPBS solution (FACS buffer, 4°C) containing 2% BSA, added to a 96-well plate with a U-shaped bottom in an amount of 5 x 10⁵ cells/100µl/well, added with the antibody diluted over concentration gradient, and incubated at 4°C for 1 hr. After centrifugation, the supernatant was discarded. 100 µl of anti-human IgG Fc-APC secondary antibody was added to each well and incubated at 4°C for 1 hr. Then the cells were washed once with a FACS buffer, and resuspended in 200 µl FACS buffer. The fluorescence signal was read on BD CantoII. The results show that ch-72C7 antibody binds to HEK293-hGPRC5D (Fig. 1) and CHOS-hGPRC5D (Fig. 2).

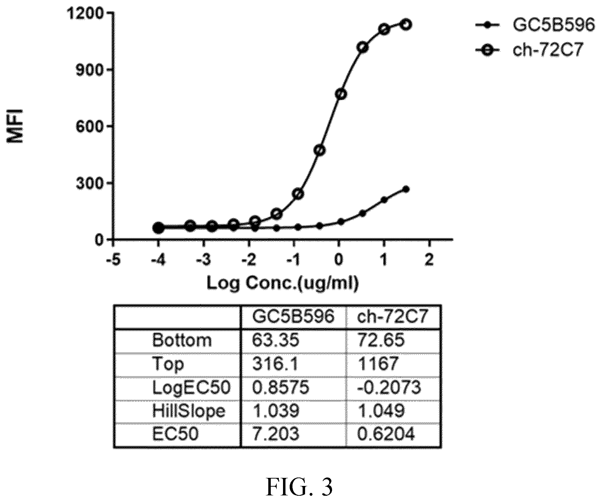

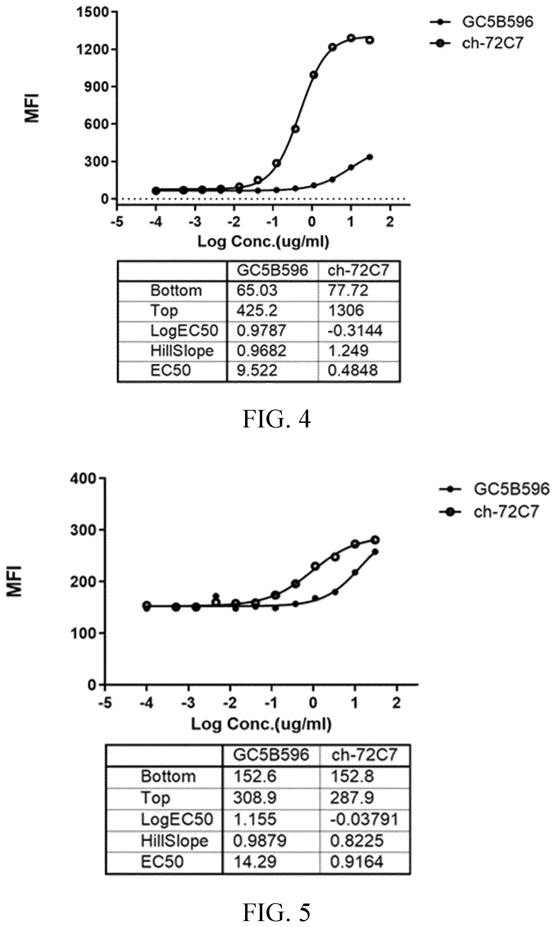

MM.1R cells, NCI-H929 cells and RPMI-8226 cells (highly expressing GPRC5D; ATCC, CL-188) were cultured in RPMI 1640 medium containing 10% FBS. After the cells were digested with TrypLE, the digested cells were centrifuged and resuspended in a DPBS solution containing 2% BSA (FACS buffer, 4°C), added into a 96-well plate with a U-shaped bottom in an amount of 5 x 10⁵ cells/100 µl/well, added with the antibody diluted over concentration gradient, and incubated at 4°C for 1 hr. After centrifugation, the supernatant was discarded. 100 µl of anti-human IgG Fc-APC secondary antibody was added to each well and incubated at 4°C for 1 hr. Then the cells were washed once with a FACS buffer, and resuspended in 200 µl FACS buffer. The fluorescence signal was read on BD CantoII. The results showed that the antibody ch-72C7 binds to MM.1R cells (Fig. 3), NCI-H929 cells (Fig. 4) and RPMI-8226 cells (Fig. 5).

**Table 7**

| Antibody | MM.1R cell binding EC50 (µg/ml) | NCI-H929 cell binding EC50 (µg/ml) | RPMI-8226 cell binding EC50 (µg/ml) |
|---|---|---|---|
| ch-72C7 | 0.620 | 0.485 | 0.916 |
| GC5B596 | 7.203 | 9.522 | 14.290 |

### Example 3: Evaluation of ADCC effect of antibodies

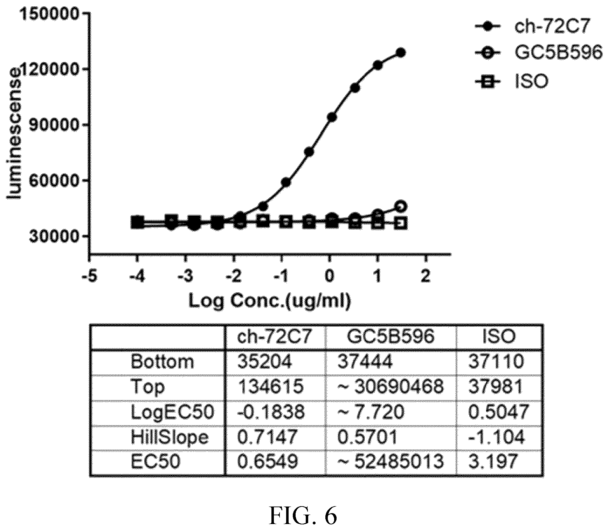

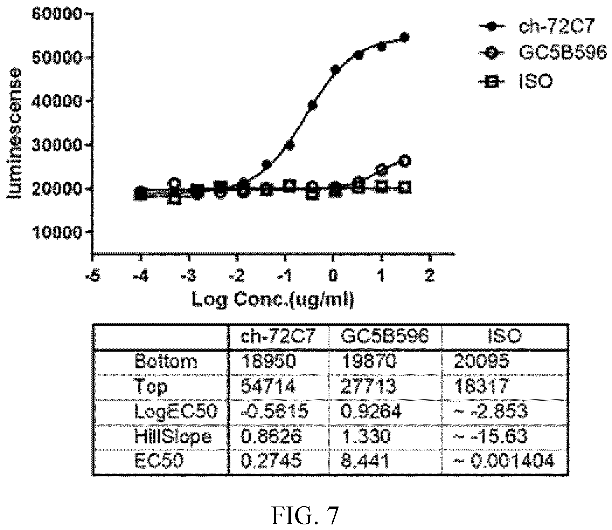

The target cells were tumor cells naturally expressing GPRC5D (NCI-H929, Nanjing Cobioer; MM.1R, Nanjing Cobioer), the effector cells were Jurkat-NFAT-Luc-CD16 cell line stably transfected with CD16 receptor and NFAT reaction element constructed by in-house, and the experiment was carried out in a 96-well flat-bottomed cell plate (Corning 3903). The antibody diluted over gradient was added to the target cells and incubated at 37°C for 30 min. 60,000 effector cells were added to every 10,000 target cells, and the reaction was carried out at 37°C for 6 hrs. After the reaction, One-Glo^{™} reagent (Promega, E6110) was added for fluorescent development, and the luminescence reading of the cell plate was measured on Tecan Spark10 plate reader. GraphPad was used for data analysis, the horizontal axis represents the logarithm of the antibody concentration, and the vertical axis represents the luminescence reading of the corresponding well. The EC50 of the antibody-dependent cytotoxicity of the anti-GPRC5D antibody was fitted from the curve. The results show that the blank control Isotype (ISO) has no cytotoxic effect on NCI-H929 cells and MM.1R cells. Both the ch-72C7 antibody and the CG5B596 antibody have cytotoxic effects on tumor cells NCI-H929 (Fig. 6) and RPMI-8226 (Fig. 7) that naturally express GPRC5D, and compared with the CG5B596 antibody, the ch-72C7 antibody shows better ADCC effect.

### Example 4: Design and expression of humanized antibodies

The ch-72C7 antibody was compared with IMGT database, a human framework sequence with the highest homology to VH/VL was selected, and CDR graft was carried out. Computational chemical simulation was carried out to maintain its binding with antigen. The amino acid sequences of the humanized antibodies have been shown in Table 8.

**Table 8**

| Humanized antibody | Sequence (the CDR region is underlined) |
|---|---|
| 22Mono5JO4 | Heavy chain variable region_VH |
| | QVQLVQSGAEVKKPGSSVKVSCRASGYPFTNYWMHWVRQAPGQ GLEWIGEINPSNGRTNYNEKFKSRATLTLDKLTSTAYMELSSLTSED TALYYCARGFAYWGQGTLVTVSS (SEQ ID NO: 15) |
| | Light chain variable region_VL |
| | EIVLTQSPGTLSLSPGERATLSCSASSSVSFMHWYQQKPGQAPRR WIYDTTKLASGIPDRFSGSGSGTDYTLTISRLEPEDFAVYYCQQWN SHPLTFGQGTTVEIK (SEQ ID NO: 14) |
| 22Mono3L7F | Heavy chain variable region_VH |
| | EVTLKESGPVLVKPTETLTLTCTASGYPFTNYWMHWVRQPPGKA LEWIGEINPSNGRTNYNEKFKSRATLSLDKSKSQAVLTMTNMDPV DTATYYCARGFAYWGQGTLVTVSS (SEQ ID NO: 17) |
| | Light chain variable region_VL |
| | EIVLTQSPATLSLSPGERATLSCSASSSVSFMHWYQQKPGQAPRRW IYDTTKLASGIPARFSGSGSGTDYTLTISSLEPEDFAVYYCQQWNSH PLTFGQGTKLEIK (SEQ ID NO: 16) |
| 22Mono4DN4 | Heavy chain variable region_VH |
| | ELVQSGAEVKKPGSSVKVSCKASGYPFTNYWMHWVRQAPGQGL EWIGEINPSNGRTNYNEKFKSRATLTLDKSTSTAYMELSSLRSEDT AVYYCARGFAYWGQGTLVTVSS (SEQ ID NO: 19) |
| | Light chain variable region_VL |
| | EIVLTQSPATLSLSPGERATLSCSASSSVSFMHWYQQKPGQAPRRW IYDTTKLASGVPARFSGSGSGTDYTLTISSLEPEDFAVYYCQQWNS HPLTFGQGTKVEIK (SEQ ID NO: 18) |
| 22Mono5UQY | Heavy chain variable region_VH |
| | QVQLQESGPGLVKPSETLSLTCTASGYPFTNYWMHWVRQPPGKG LEWIGEINPSNGRTNYNEKFKSRATLSLDKSKNQASLNLDSVSAA DTAIYYCARGFAYWGKGSTVTVSS (SEQ ID NO: 21) |
| | Light chain variable region_VL |
| | AIQLTQSPSSLSASVGDRVTITCSASSSVSFMHWYQQKPGKAPKR WIYDTTKLASGVPSRFSGSGSGTDYTFTISSLQPEDIATYYCQQWN SHPLTFGPGTKVDIK (SEQ ID NO: 20) |

The VH and VL regions of the above antibody were linked to the human IgG1 Fc region and kappa light chain constant region, and the heavy chain and light chain sequences of the antibody were inserted into the pcDNA3.4 vector. The vector was transiently expressed in HEK293 cells, and the antibody was purified by protein A or G.

Moreover, VH and VL of ch-72C7 antibody were substituted for VH and VL of human IgG1 antibody, respectively, to form chimeric antibody 22mono, which was used as a control to evaluate the humanization result of each antibody.

**Table 9**

| Chimeric antibody | Sequence (the CDR region is underlined) |
|---|---|
| 22Mono | QVQLQQPGAELVKPGTSVKLSCKASGYPFTNYWMHWVRQRPGQGLE WIGEINPSNGRTNYNEKFKSLATLTLDKSSSTAYMHLNSLTSEDSAVYYC ARGFAYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYI CNVNHKPSNTKVDKKVEPKSCDKTHTCPPCPAPELLGGPSVFLFPPKPK DTLMISRTPEVTCVVVDVSHEDPEVKFNWYVDGVEVHNAKTKPREEQ YNSTYRVVSVLTVLHQDWLNGKEYKCKVSNKALPAPIEKTISKAKGQP REPQVYTLPPSRDELTKNQVSLTCLVKGFYPSDIAVEWESNGQPENNYK TTPPVLDSDGSFFLYSKLTVDKSRWQQGNVFSCSVMHEALHNHYTQKS LSLSP (SEQ ID NO: 26) Heavy chain_HC |
| | QIVLSQFPAIMSASPGEKVTMTCSASSSVSFMHWYQQKSGTSPKRWIYD TTKLASGVPARFTGSGSGTSYSLTISSMEAEDAATYYCQQWNSHPLTFG Light chain_LC |
| | PGTKLELKRTVAAPSVFIFPPSDEQLKSGTASVVCLLNNFYPREAKVQW KVDNALQSGNSQESVTEQDSKDSTYSLSSTLTLSKADYEKHKVYACEV THQGLSSPVTKSFNRGEC (SEQ ID NO: 27) |

### Example 5: Affinity test of humanized GPRC5D antibody

The binding affinity of the humanized antibody obtained by recombinant expression through the method in Example 4 to human GPRC5D was analyzed. The recombinant GPRC5D antigen (Human GPRC5D protein-Flag-His tag (51.8KD) ACRO Cat:GPD-H52D3) was immobilized on a chip and detected by Octet^{®} R8 biomolecular interaction analysis system. The results are shown in Table 10.

**Table 10**

| Test sample | KD (M) | kₒₙ(1/Ms) | k_{dis}(1/s) |
|---|---|---|---|
| 22Mono5JO4 | 7.87E-10 | 1.51E+05 | 1.19E-04 |
| 22monoO3L7 | 2.56E-08 | 7.80E+04 | 2.00E-03 |
| 22mono4DN4 | 1.69E-08 | 6.73E+04 | 1.14E-03 |
| 22mono5UQY | 5.67E-09 | 8.62E+04 | 4.89E-04 |

The results show that the four humanized GPRC5D antibodies all have excellent binding affinity to human GPRC5D antigen, and 22Mono5JO4 has the strongest binding affinity to the antigen. The above-mentioned humanized antibodies have better binding affinity to the human GPRC5D antigen than the original mouse antibody ch-72C7. Therefore, it is expected that the binding affinity to human GPRC5D antigen and the cytotoxicity to disease cells expressing human GPRC5D antigen are at least comparable to or even better than that of mouse antibody ch-72C7.

Although the present application has been particularly shown and described with reference to specific embodiments (some of which are preferred embodiments), it will be understood by those skilled in the art that various changes in form and details can be made without departing from the spirit and scope of the present application disclosed herein.

## Claims

1. An anti-GPRC5D antibody or an antigen binding fragment thereof, comprising three heavy chain complementarity determining regions HCDR1, HCDR2 and HCDR3, and three light chain complementarity determining regions LCDR1, LCDR2 and LCDR3, wherein the heavy chain complementarity determining regions are identical to three heavy chain complementarity determining regions comprised in a heavy chain variable region (V_{H}) as shown in SEQ ID NO: 13, and the light chain complementarity determining regions are identical to three light chain complementarity determining regions comprised in a light chain variable region (V_{L}) as shown in SEQ ID NO: 12.

2. An anti-GPRC5D antibody or an antigen binding fragment thereof, comprising three heavy chain complementarity determining regions HCDR1, HCDR2 and HCDR3, and three light chain complementarity determining regions LCDR1, LCDR2 and LCDR3, wherein
a) the HCDR1 comprises an amino acid sequence selected from the group consisting of SEQ ID NO: 4 or a variant thereof having no more than 3, 2 or 1 amino acid substitution, or SEQ ID NO: 9 or a variant thereof having no more than 3, 2 or 1 amino acid substitution;
b) the HCDR2 comprises an amino acid sequence selected from the group consisting of SEQ ID NO: 5 or a variant thereof having no more than 3, 2 or 1 amino acid substitution, or SEQ ID NO: 10 or a variant thereof having no more than 3, 2 or 1 amino acid substitution;
c) the HCDR3 comprises an amino acid sequence selected from the group consisting of SEQ ID NO: 6 or a variant thereof having no more than 3, 2 or 1 amino acid substitution, or SEQ ID NO: 11 or a variant thereof having no more than 3, 2 or 1 amino acid substitution;
d) the LCDR1 comprises an amino acid sequence selected from the group consisting of SEQ ID NO: 1 or a variant thereof having no more than 3, 2 or 1 amino acid substitution, or SEQ ID NO: 7 or a variant thereof having no more than 3, 2 or 1 amino acid substitution;
e) the LCDR2 comprises an amino acid sequence selected from the group consisting of SEQ ID NO: 2 or a variant thereof having no more than 3, 2 or 1 amino acid substitution, or SEQ ID NO: 8 or a variant thereof having no more than 3, 2 or 1 amino acid substitution; and
f) the LCDR3 comprises an amino acid sequence selected from the group consisting of SEQ ID NO: 3 or a variant thereof having no more than 3, 2 or 1 amino acid substitution.

3. The antibody or antigen binding fragment thereof according to any one of preceding claims, wherein
a) the HCDR1 comprises the amino acid sequence as shown in SEQ ID NO: 4 or a variant thereof having no more than 3, 2 or 1 amino acid substitution, the HCDR2 comprises the amino acid sequence as shown in SEQ ID NO: 5 or a variant thereof having no more than 3, 2 or 1 amino acid substitution, the HCDR3 comprises the amino acid sequence as shown in SEQ ID NO: 6 or a variant thereof having no more than 3, 2 or 1 amino acid substitution, the LCDR1 comprises the amino acid sequence as shown in SEQ ID NO: 1 or a variant thereof having no more than 3, 2 or 1 amino acid substitution, the LCDR2 comprises the amino acid sequence as shown in SEQ ID NO: 2 or a variant thereof having no more than 3, 2 or 1 amino acid substitution, and the LCDR3 comprises the amino acid sequence as shown in SEQ ID NO: 3 or a variant thereof having no more than 3, 2 or 1 amino acid substitution; or
b) the HCDR1 comprises the amino acid sequence as shown in SEQ ID NO: 9 or a variant thereof having no more than 3, 2 or 1 amino acid substitution, the HCDR2 comprises the amino acid sequence as shown in SEQ ID NO: 10 or a variant thereof having no more than 3, 2 or 1 amino acid substitution, the HCDR3 comprises the amino acid sequence as shown in SEQ ID NO: 11 or a variant thereof having no more than 3, 2 or 1 amino acid substitution, the LCDR1 comprises the amino acid sequence as shown in SEQ ID NO: 7 or a variant thereof having no more than 3, 2 or 1 amino acid substitution, the LCDR2 comprises the amino acid sequence as shown in SEQ ID NO: 8 or a variant thereof having no more than 3, 2 or 1 amino acid substitution, and the LCDR3 comprises the amino acid sequence as shown in SEQ ID NO: 3 or a variant thereof having no more than 3, 2 or 1 amino acid substitution.

4. The antibody or antigen binding fragment thereof according to any one of preceding claims, wherein the heavy chain variable region comprises the amino acid sequence as shown in SEQ ID NO: 13 or a variant thereof having no more than 3, 2 or 1 amino acid substitution, and the light chain variable region comprises the amino acid sequence as shown in SEQ ID NO: 12 or a variant thereof having no more than 3, 2 or 1 amino acid substitution.

5. The antibody or antigen binding fragment thereof according to any one of preceding claims, wherein the antibody or antigen binding fragment thereof is humanized.

6. The antibody or antigen binding fragment thereof according to any one of preceding claims, wherein the antibody or antigen binding fragment thereof comprises a heavy chain variable region (V_{H}) and/or a light chain variable region (V_{L}); and
a) the heavy chain variable region comprises an amino acid sequence selected from the group consisting of SEQ ID NO: 15, SEQ ID NO: 17, SEQ ID NO: 19, and SEQ ID NO: 21, or a variant thereof having no more than 3, 2 or 1 amino acid substitution; and
b) the light chain variable region comprises an amino acid sequence selected from the group consisting of SEQ ID NO: 14, SEQ ID NO: 16, SEQ ID NO: 18, and SEQ ID NO: 20, or a variant thereof having no more than 3, 2 or 1 amino acid substitution.

7. The antibody or antigen binding fragment thereof according to any one of preceding claims, wherein the antibody or antigen binding fragment thereof comprises a heavy chain variable region (V_{H}) and/or a light chain variable region (V_{L}); and
a) the heavy chain variable region comprises the amino acid sequence as shown in SEQ ID NO: 15 or a variant thereof having no more than 3, 2 or 1 amino acid substitution, and the light chain variable region comprises the amino acid sequence as shown in SEQ ID NO: 14 or a variant thereof having no more than 3, 2 or 1 amino acid substitution,
b) the heavy chain variable region comprises the amino acid sequence as shown in SEQ ID NO: 17 or a variant thereof having no more than 3, 2 or 1 amino acid substitution, and the light chain variable region comprises the amino acid sequence as shown in SEQ ID NO: 16 or a variant thereof having no more than 3, 2 or 1 amino acid substitution;
c) the heavy chain variable region comprises the amino acid sequence as shown in SEQ ID NO: 19 or a variant thereof having no more than 3, 2 or 1 amino acid substitution, and the light chain variable region comprises the amino acid sequence as shown in SEQ ID NO: 18 or a variant thereof having no more than 3, 2 or 1 amino acid substitution; or
d) the heavy chain variable region comprises the amino acid sequence as shown in SEQ ID NO: 21 or a variant thereof having no more than 3, 2 or 1 amino acid substitution, and the light chain variable region comprises the amino acid sequence as shown in SEQ ID NO: 20 or a variant thereof having no more than 3, 2 or 1 amino acid substitution,
wherein the variant still retains the specific binding affinity to GPRC5D.

8. The antibody or antigen binding fragment thereof according to claim 7, wherein the amino acid substitution does not occur in the CDR region.

9. The antibody or antigen binding fragment thereof according to any one of preceding claims, which is a diabody, Fab, Fab', F(ab')2, Fd, a Fv fragment, a disulfide-stabilized Fv fragment (dsFv), (dsFv)2, a bispecific dsFv (dsFv-dsFv'), a disulfide-stabilized diabody (ds diabody), a single-chain antibody molecule (scFv), or a scFv dimer (a divalent diabody).

10. The antibody or antigen binding fragment thereof according to any one of preceding claims, further comprising an immunoglobulin constant region, optionally a human immunoglobulin constant region, or optionally a human IgG constant region.

11. The antibody or antigen binding fragment thereof according to claim 9, wherein the human IgG constant region is derived from IgG1, IgG2, IgG3 or IgG4.

12. The antibody or antigen binding fragment thereof according to claim 9 or 10, wherein the constant region comprises one or more amino acid residue substitutions or modifications which lead to increased CDC or ADCC relative to wild-type constant region.

13. The antibody or antigen binding fragment thereof according to any one of preceding claims, which is bispecific or multi-specific.

14. A nucleic acid, comprising a nucleotide sequence encoding the antibody or antigen binding fragment thereof according to any one of claims 1 to 13.

15. A vector, comprising the nucleic acid according to claim 14.

16. A host cell, comprising the nucleic acid according to claim 14 or the vector according to claim 15.

17. A conjugate, comprising the antibody or antigen binding fragment thereof according to any one of claims 1 to 13 and a payload conjugated thereto, wherein the payload is selected from the group consisting of a radioactive label, a fluorescent label, an enzyme substrate label, an affinity purification tag, a tracer molecule, an anticancer drug and a cytotoxic molecule.

18. A chimeric antigen receptor (CAR), comprising an antigen binding domain, a transmembrane domain, and an immune cell signal transduction domain, wherein the antigen binding domain specifically binds to GPRC5D and comprises the antigen binding fragment according to any one of claims 1 to 13.

19. A nucleic acid, encoding the chimeric antigen receptor (CAR) according to claim 18.

20. A cell, comprising a nucleic acid sequence according to claim 19.

21. A genetically modified cell expressing the CAR according to claim 19.

22. A pharmaceutical composition, comprising the antibody or antigen binding fragment thereof according to any one of claims 1 to 13, the nucleic acid according to claim 14, the vector according to claim 15, the conjugate according to claim 18, the nucleic acid according to claim 19, or the cell according to claim 20 or 21, and a pharmaceutically acceptable carrier.

23. A method for treating or preventing a disease, a condition, or a disorder, comprising administering a therapeutically effective amount of the antibody or antigen binding fragment thereof according to any one of claims 1 to 13, the nucleic acid according to claim 14, the vector according to claim 15, the conjugate according to claim 18, the nucleic acid according to claim 19, the cell according to claim 20 or 21, or the composition according to claim 22 to a subject in need thereof.

24. The method according to claim 23, wherein the disease, the condition or the disorder is selected from the group consisting of cancers, autoimmune diseases, and inflammations.

25. A method for stimulating an immune cell (such as T cell or NK cell)-mediated immune response to cells or tissues expressing GPRC5D in a mammal, the method comprising administering an effective amount of genetically modified cells to the mammal to express the CAR according to claim 18.

26. A method for treating a mammal having a GPRC5D-related disease or condition, the method comprising administering an effective amount of the cell according to claim 20 to the mammal, to treat the mammal.

27. The method according to claim 26, wherein the cell is autologous T cells or autologous NK cells.
